# EUROPEAN PATENT SPECIFICATION

(11) **EP 2 298 925 B1**
(45) Date of publication and mention of the grant of the patent: **20.02.2013**
(21) Application number: 09757650.8
(22) Date of filing: 01.06.2009
(51) Int. Cl.: C12N 15/861

(54) **SYSTEM FOR PACKAGING OF HIGH-CAPACITY ADENOVIRUSES**
SYSTEM ZUR VERPACKUNG VON ADENOVIREN HOHER KAPAZITÄT
SYSTÈME D'EMPAQUETAGE D'ADÉNOVIRUS DE GRANDE CAPACITÉ

(30) Priority: 04.06.2008 ES 200801682
(43) Date of publication of application: 23.03.2011
(73) Proprietor: Proyecto de Biomedicina Cima, S.L., 31008 Pamplona - Navarra (ES)
(72) Inventor: QIAN, Cheng, E-31008 Pamplona - Navarra (ES); MOURIÑO LÓPEZ, Susana, E-31008 Pamplona - Navarra (ES); PRIETO VALTUEÑA, Jesús María, E-31008 Pamplona - Navarra (ES)
(74) Representative: ABG Patentes, S.L.
(86) International application number: PCT/ES2009/070194
(87) International publication number: WO 2009/147271

(56) References cited:
- WO-A-02/092786
- NICOLAS A L ET AL: "Creation and Repair of Specific DNA Double-Strand Breaks in Vivo Following Infection with Adenovirus Vectors Expressing Saccharomyces cerevisiae HO Endonuclease" VIROLOGY, ACADEMIC PRESS,ORLANDO, US, vol. 266, no. 1, 5 January 2000 (2000-01-05), pages 211-224, XP004436199 ISSN: 0042-6822
- BURCIN ET AL: "Adenovirus-mediated regulable target gene expression in vivo" PROCEEDINGS OF THE NATIONAL ACADEMY OF SCIENCES OF USA, NATIONAL ACADEMY OF SCIENCE, WASHINGTON, DC, US, vol. 96, 1 January 1999 (1999-01-01), pages 355-360, XP002130326 ISSN: 0027-8424
- MILLS KEVIN D ET AL: "MEC1-dependent redistribution of the Sir3 silencing protein from telomeres to DNA double-strand breaks" CELL, CELL PRESS, CAMBRIDGE, NA, US, vol. 97, no. 5, 28 May 1999 (1999-05-28), pages 609-620, XP002312631 ISSN: 0092-8674
- BEERLI ROGER R ET AL: "Chemically regulated zinc finger transcription factors" JOURNAL OF BIOLOGICAL CHEMISTRY, AMERICAN SOCIETY OF BIOLOCHEMICAL BIOLOGISTS, BIRMINGHAM, US, vol. 275, no. 42, 20 October 2000 (2000-10-20), pages 32617-32627, XP002199787 ISSN: 0021-9258
- HILLGENBERG M ET AL: "SYSTEM FOR EFFICIENT HELPER-DEPENDENT MINIMAL ADENOVIRUS CONSTRUCTION AND RESCUE" HUMAN GENE THERAPY, MARY ANN LIEBERT, NEW YORK ,NY, US, vol. 12, no. 6, 10 April 2001 (2001-04-10) , pages 643-657, XP009002050 ISSN: 1043-0342
- GAO G ET AL: "High throughput creation of recombinant adenovirus vectors by direct cloning, green-white selection and I-Sce I-mediated rescue of circular adenovirus plasmids in 293 cells" GENE THERAPY, MACMILLAN PRESS LTD., BASINGSTOKE, GB, vol. 10, no. 22, 1 October 2003 (2003-10-01), pages 1926-1930, XP002405401 ISSN: 0969-7128
- ANGLANA MAURO ET AL: "Construction of a recombinant adenovirus for efficient delivery of the I-SceI yeast endonuclease to human cells and its application in the in vivo cleavage of chromosomes to expose new potential telomeres" NUCLEIC ACIDS RESEARCH, OXFORD UNIVERSITY PRESS, SURREY, GB, vol. 27, no. 21, 1 November 1999 (1999-11-01), pages 4276-4281, XP002155138 ISSN: 0305-1048
- GOUBLE AGNÈS ET AL: "Efficient in toto targeted recombination in mouse liver by meganuclease-induced double-strand break" JOURNAL OF GENE MEDICINE, WILEY, US, vol. 8, no. 5, 1 May 2006 (2006-05-01), pages 616-622, XP002427714 ISSN: 1099-498X

### SEQUENCE LISTING

<110> PROYECTO DE BIOMEDICINA CIMA S.L.
<120> SYSTEM FOR PACKAGING OF HIGH-CAPACITY ADENOVIRUS
<130> P3918PC00
<150> P200801682
   <151> 2008-06-04
<160> 48
<170> PatentIn version 3.5
<210> 1
   <211> 17
   <212> DNA
   <213> Artificial sequence
<220>
   <223> Sequence recognized by the DNA binding domain of GAL4
<220>
   <221> c
   <222> (1)..(1)
<400> 1
   tggagtactg tcctccg 17
<210> 2
   <211> 69
   <212> DNA
   <213> Artificial sequence
<220>
   <223> Linker RU-1
<400> 2
   ggaccaccaa ggatcctcta gaaccgttta aacaccacta gtccaacgcg tgatatcacc 60
   gcggccgca 69
<210> 3
   <211> 77
   <212> DNA
   <213> Artificial sequence
<220>
   <223> Linker RU-2
<400> 3
   gatctgcggc cgcggtgata tcacgcgttg gactagtggt gtttaaacgg ttctagagga 60
   tccttggtgg tcctgca 77
<210> 4
   <211> 20
   <212> DNA
   <213> Artificial sequence
<220>
   <223> Cebador Ad19
<400> 4
   gagtgccagc gagtagagtt 20
<210> 5
   <211> 24
   <212> DNA
   <213> Artificial sequence
<220>
   <223> Ad22
<400> 5
   gaattctcaa tctgtatctt catc 24
<210> 6
   <211> 21
   <212> DNA
   <213> Artificial sequence
<220>
   <223> SceI-b1 Primer
<400> 6
   aaaccatggg atcaagatcg c 21
<210> 7
   <211> 33
   <212> DNA
   <213> Artificial sequence
<220>
   <223> SceI-b2 Primer
<400> 7
   ccctctagat tatttcagga aagtttcgga gga 33
<210> 8
   <211> 28
   <212> DNA
   <213> Artificial sequence
<220>
   <223> PRU-1 Primer
<400> 8
   aatactagtc cgagctctta cgcgggtc 28
<210> 9
   <211> 31
   <212> DNA
   <213> Artificial sequence
<220>
   <223> PRU-2 Primer
<400> 9
   aatacgcgtg gtggcctgtg aagagaaaaa a 31
<210> 10
   <211> 17
   <212> DNA
   <213> Artificial sequence
<220>
   <223> Target sequence of the SceI endonuclease
<400> 10
   tagggataac agggtaa 17
<210> 11
   <211> 36
   <212> DNA
   <213> Artificial sequence
<220>
   <223> attB-P1 Primer
<400> 11
   agatctgtcg acgatgtagg tcacggtctc cgaagc 36
<210> 12
   <211> 44
   <212> DNA
   <213> Artificial sequence
<220>
   <223> attb-P2 primer
<400> 12
   ttaattaatt accctgttat ccctagtcga catgcccgcc gtga 44
<210> 13
   <211> 43
   <212> DNA
   <213> Artificial sequence
<220>
   <223> Ad63 Primer
<400> 13
   agatctaggg ataacagggt aatcatcatc aataatatac ctt 43
<210> 14
   <211> 26
   <212> DNA
   <213> Artificial sequence
<220>
   <223> ad64 primer
<400> 14
   agatctcggc gcacaccaaa aacgtc 26
<210> 15
   <211> 26
   <212> DNA
   <213> Artificial sequence
<220>
   <223> ad57 primer
<400> 15
   agatctcgtg gcttaagggt gggaaa 26
<210> 16
   <211> 28
   <212> DNA
   <213> Artificial sequence
<220>
   <223> ad58 primer
<400> 16
   tctagattat atggctggga acatagcc 28
<210> 17
   <211> 28
   <212> DNA
   <213> Artificial sequence
<220>
   <223> ad59 primer
<400> 17
   tctagattag ctatgctaac cagcgtag 28
<210> 18
   <211> 44
   <212> DNA
   <213> Artificial sequence
<220>
   <223> ad65 primer
<400> 18
   ggtacctagg gataacaggg taatcatcat caataatata cctt 44
<210> 19
   <211> 21
   <212> DNA
   <213> Artificial sequence
<220>
   <223> IIIa1 primer
<400> 19
   agagccagcc gtccggcctt a 21
<210> 20
   <211> 21
   <212> DNA
   <213> Artificial sequence
<220>
   <223> IIIa2 primer
<400> 20
   cgcggtcgcc tgtgggagcc c 21
<210> 21
   <211> 20
   <212> DNA
   <213> Artificial sequence
<220>
   <223> IIIa3 primer
<400> 21
   ggttgcacta aacgccttcc 20
<210> 22
   <211> 20
   <212> DNA
   <213> Artificial sequence
<220>
   <223> IIIa4 primer
<400> 22
   agcccctgca agtttttgaa 20
<210> 23
   <211> 21
   <212> DNA
   <213> Artificial sequence
<220>
   <223> IV1 primer
<400> 23
   cctctagtta cctccaatgg c 21
<210> 24
   <211> 21
   <212> DNA
   <213> Artificial sequence
<220>
   <223> IV2 primer
<400> 24
   agtaacttta gtttgcaagg a 21
<210> 25
   <211> 20
   <212> DNA
   <213> Artificial sequence
<220>
   <223> IV3 primer
<400> 25
   gggcaacaca ctcaccatgc 20
<210> 26
   <211> 20
   <212> DNA
   <213> Artificial sequence
<220>
   <223> IV4 primer
<400> 26
   acatgcaaag gagccccgta 20
<210> 27
   <211> 21
   <212> DNA
   <213> Artificial sequence
<220>
   <223> Pr1 primer
<400> 27
   acagctgccg ccatgggctc c 21
<210> 28
   <211> 21
   <212> DNA
   <213> Artificial sequence
<220>
   <223> Pr2 primer
<400> 28
   gggcgagtgg cgctccagga a 21
<210> 29
   <211> 19
   <212> DNA
   <213> Artificial sequence
<220>
   <223> Pr3 primer
<400> 29
   gcttttctga ccagcgact 19
<210> 30
   <211> 20
   <212> DNA
   <213> Artificial sequence
<220>
   <223> Pr4 primer
<400> 30
   cactgtggct gcggaagtag 20
<210> 31
   <211> 21
   <212> DNA
   <213> Artificial sequence
<220>
   <223> POL1 primer
<400> 31
   ctggcctgga cgcgagcctt t 21
<210> 32
   <211> 21
   <212> DNA
   <213> Artificial sequence
<220>
   <223> Cebador POL2
<400> 32
   cagggtcctc gtcagcgtag t 21
<210> 33
   <211> 21
   <212> DNA
   <213> Artificial sequence
<220>
   <223> POL3 primer
<400> 33
   tacggggaca cggacagcct t 21
<210> 34
   <211> 21
   <212> DNA
   <213> Artificial sequence
<220>
   <223> POL4 primer
<400> 34
   cttggcgcgc agcttgccct t 21
<210> 35
   <211> 20
   <212> DNA
   <213> Artificial sequence
<220>
   <223> PRC1 primer
<400> 35
   tgatccagtc gtagcaggag 20
<210> 36
   <211> 20
   <212> DNA
   <213> Artificial sequence
<220>
   <223> PRC4 primer
<400> 36
   acgatctcat cctggaacac 20
<210> 37
   <211> 21
   <212> DNA
   <213> Artificial sequence
<220>
   <223> III1 primer
<400> 37
   gccagtggcg gcggcgctgg g 21
<210> 38
   <211> 21
   <212> DNA
   <213> Artificial sequence
<220>
   <223> III2 primer
<400> 38
   ccgatgtcgc tttccagaac c 21
<210> 39
   <211> 21
   <212> DNA
   <213> Artificial sequence
<220>
   <223> E4-1 primer
<400> 39
   cgtgcgaggt cttccctgca g 21
<210> 40
   <211> 21
   <212> DNA
   <213> Artificial sequence
<220>
   <223> E4-2 primer
<400> 40
   ctacatgggg gtagagtcat a 21
<210> 41
   <211> 21
   <212> DNA
   <213> Artificial sequence
<220>
   <223> RT-EA1 primer
<400> 41
   tatctgccac ggaggtgtta t 21
<210> 42
   <211> 21
   <212> DNA
   <213> Artificial sequence
<220>
   <223> RT-EA2 primer
<400> 42
   tagacaaaca tgccacaggt c 21
<210> 43
   <211> 21
   <212> DNA
   <213> Artificial sequence
<220>
   <223> RT-EB3 primer
<400> 43
   gcttgggagt gtttggaaga t 21
<210> 44
   <211> 21
   <212> DNA
   <213> Artificial sequence
<220>
   <223> RT-EB4 primer
<400> 44
   ctgctcctcc gtcggtatta t 21
<210> 45
   <211> 20
   <212> DNA
   <213> Artificial sequence
<220>
   <223> SceI-3 primer
<400> 45
   ccatacgatg ttcctgacta 20
<210> 46
   <211> 21
   <212> DNA
   <213> Artificial sequence
<220>
   <223> SceI-4 primer
<400> 46
   cccttaacca ggtattctac t 21
<210> 47
   <211> 21
   <212> DNA
   <213> Artificial sequence
<220>
   <223> Gal-1 primer
<400> 47
   ctgactaggg cacatctgac a 21
<210> 48
   <211> 21
   <212> DNA
   <213> Artificial sequence
<220>
   <223> Gal-2 primer
<400> 48
   ttgatgagct ctctaatgta g 21

## Description

### TECHNICAL FIELD OF THE INVENTION

The invention relates to the field of biotechnology and, more specifically, to the field of vectors for the expression of heterologous genes in target cells. In particular, the invention relates to a system for the production and packaging of high-capacity adenoviral vectors useful for gene transfer.

### BACKGROUND

High-capacity adenovirus (HC), also known as *gutless* adenovirus or *helper*-dependent adenovirus, are highly attractive as *in vivo* vectors for gene therapy as they show a very reduced associated immunogenicity and have a high transduction efficiency and wide tropism, both in rodents and in primates. These vectors are characterized in that they only contain the DNA sequences necessary for their replication and packaging and lack the other adenoviral genes, which results in that it is possible to accommodate sequences of up to 36 kb. For the replication of the viral DNA and its packaging in viral particles, the only adenoviral sequences required in *cis* by these vectors are the ITR (*inverted terminal repeats*) sequences, located at both ends of the linear DNA, and the packaging signal (ψ) (Grable and Hearing, 1992, *J. Virol.,* 66: 723-731; Hearing and Shenk, 1983, *Cell,* 33: 695-703).

Since high-capacity adenovirus lack all the viral coding regions, their propagation is only possible if the cells are co-infected with a second virus called *helper* or auxiliary virus, i.e. a virus capable of encoding all the proteins necessary for the replication of a defective virus and its encapsidation in viral particles with infective capacity. In the case of the production of high-capacity adenoviral vectors, the *helper* virus is characterized in that it has a deletion in the E1 adenoviral region and in that it provides, *in trans,* the whole repertoire of viral proteins necessary for the replication and assembly of the progeny of the *gutless* virus. The propagation of the latter requires the use of cell lines capable of complementing the deletion of said E1 region in the *helper* virus, such as 293 line (Graham, *et al.,* 1977, *J*. *Gene. Virol.,* 36: 59-74) or the PerC6 line (Fallaux *et al.,* 1998, *Hum. Gene Ther*., 9: 1909-1917).

Alternatively, Gao *et al.* (2003, *Gene Ther*., 10: 1926-1930) discloses the *in vivo* rescue of adenoviruses by endonuclease linearization. In detail, a circular plasmid was engineered containing the entire adenoviral genome, with a prokaryotic expression cassette containing GFP that replaced E1a and E1b and two I-SceI recognition sites outside the ITRs. The plasmid was used to transfect 293 cells expressing the endonuclease I-SceI (which provide protein E1 in trans). Alternatively, the I-SceI expression cassette is provided in the circular plasmid. The linear virus is rescued through an enzymatic reaction mediated by the endonuclease I-*Sce*I. Thus, the resulting replication defective virus lacks E1 and is flanked by restriction sites.

However, this propagation system also results in the generation of undesirable viral particles which encapsidate genomes of *helper* virus and contaminate the final preparations of the high-capacity adenovirus. To date, different systems have been developed and described to try to eliminate said contaminations blocking the packaging process of the *helper* virus. In general, these systems are based on the incorporation of mutations in the packaging signal of the *helper* virus, in the increase in size of its genome and, more particularly, in a specific elimination of the packaging signal during the viral production process.

WO02092786A2 discloses an adenoviral helper vector which contains and a single target site for an endonuclease. When the adenoviral helper vector is expressed in a mammalian cell which expresses the endonuclease, the adenoviral genome is cleaved by the action of the endonuclease, thus resulting in two genomic fragments which cannot be packaged. All the genes necessary for the processes of replication and packaging of the viral particles are included within the sequence of the adenoviral vector.

Parks *et al.* (1996, *Proc. Natl. Acad. Sci. USA,* 93: 13565-13570) have described the elimination of the ψ packaging signal of the *helper* virus by a Cre-loxP recombination system. In this system, the ψ signal is flanked by loxP sites and the propagation of the virus is carried out in 293 cells which express the Cre recombinase (293Cre cells). When the *helper* virus enters the cell, the recombinase excises the ψ signal preventing it from being able to be packaged but maintaining its replication capacity and, therefore, the expression of the encoding sequences necessary for the packaging of the high-capacity adenovirus.

*Groth et al. (Proc. Natl. Acad. Sci. USA,* 2000, 97: 5995-6000) have proposed the use of a unidirectional recombinase, in particular the Φ C31 recombinase, wherein *attB*/*attP* sites specificically recognised are placed flanking the ψ signal. When PhiC31 recombinase excises the sequence flanked by the *attB*/*attP* sites, it modifies the recognition sequences and converts them into *attR*/*attL* sequences preventing the recognition from occurring again and, therefore, the reintroduction of the ψ signal.

Ng *et al.* (2001, *Mol. Ther.,* 3: 809-815) have proposed an alternative recombination system FLP/frt, wherein the propagation of the high-capacity adenovirus is carried out in 293 cells capable of expressing the yeast FLP recombinase (293FLP and 293CreFLP line) and wherein the ψ signal of the *helper* virus is flanked by frt sites specifically recognized by said FLP recombinase. The amplification efficiency of the *gutless* virus (10⁸ bfu/ml in run 3), as well as the contamination levels *of helper* virus (0.5% after purification in CsCl gradient), are very similar to those produced with the previous Cre-loxP system.

In order to attempt to improve these systems, Palmer and Ng (2003, *Mol. Ther.,* 8: 846-852) have developed a system derived from the Cre-loxP system, where the inversion of the packaging signal of the *helper* virus has been included.

On the other hand, Sargent *et al.* (2004, *Gene Ther.,* 11: 504-511) have proposed a size-based restriction system, wherein a *helper* virus of more than 35 kb is used and deleted in the gene of the IX protein, as well as a 293 cell line capable of complementing said deletion.

However, despite the improvements, to date none of these systems has managed to avoid recombinations between the sequences of the two virus included in the production process and, consequently, the contamination of the sample with replicative adenovirus. In a recent review Alba *et al.* (2005, *Gene Ther.,* 12 Suppl 1: S18-27) state that the contamination levels may oscillate, depending on the system used, between 0.02% and 1% with respect to the number of viral particles of high-capacity adenovirus produced. These contamination levels are considered too high for their possible use in clinical procedures of gene therapy.

Additionally, another important limitation for the use of high capacity adenovirus is related to the difficulty to produce them on a large scale. Currently, this type of production is complex, has low efficiency and requires numerous successive cycles of co-infection with the *helper* virus.

To do this, it is necessary to improve the procedures while at the same time developing alternative systems to produce high-capacity adenoviral vectors in the quantities and with the quality (free from contaminating viral particles) required for their clinical use.

The ideal solution to this problem would be the development of a specific cell line for the large scale production of high-capacity adenoviral vectors. However, the production systems independent of the participation of a *helper* virus pose the problem of the cytotoxicity derived from high expression levels of the adenoviral genes which, to date, have enormously limited the development of this type of production systems. To this, we have to add the fact that it is extremely complicated to reproduce the correct sequence of the events necessary to coordinate the replication of DNA with the expression of the structural proteins which permit the mass production of viral particles during the late stage of the infection (Farley, *et al.,* 2004, *J*. *Virol.,* 78: 1782-1791).

WO0072887 discloses a high-capacity adenovirus production system independent of *helper* adenovirus based on a binary system formed by (i) a packaging cell line which comprises in stable form a circular episome in a reduced number of copies and based on replicative elements of the Epstein-Barr virus (EBV) together with an adenoviral genome deleted in regions E1, E3, E4 and in genes pTP and DBP, and (ii) a vector which comprises the elements necessary for the replication and packaging of the adenoviral genome and which are absent in the episome (E4 region, pTP gene and DBP) as well as the heterologous gene. In this system the episome is not packaged in virions since (i) it lacks the packaging signal and (ii) its size exceeds the packaging capacity of the adenovirus.

On the other hand, WO0042208 discloses an adenovirus production system independent of *helper* virus which comprises (i) an adenoviral vector deleted in regions E1, E3 and the gene that encodes the protein of the fibre (L5), which is involved in the assembly process of the virions, and (ii) a specific cell line capable of complementing the replication and the packaging of said vector. All the genes necessary for the processes of replication and packaging of the viral particles are included within the sequence of the adenoviral vector.

Finally, the system recently described by Catalucci *et al.* (2005, *J*. *Virol.,* 79: 6400-6409) suggests the use of an episomal plasmid composed of the origin of replication of the Epstein-Barr virus, which permits its maintenance in the nucleus of a 293 cell line modified for the stable expression of the nuclear antigen EBNA1 (293EBNA). This episome includes the ITR sequences of the serotype 5 adenovirus, the genes of the early region (E2) and ORF6 included in the E4 region of the adenoviral genome. The transcription of these adenoviral genes are under the control of an inducible promoter whilst the other genes necessary for the formation of the capsid are included within the sequence of the high-capacity adenovirus.

In general, all these helper-independent adenoviral vector production systems are limited by the size of the DNA sequence that can be inserted in the vector, since all these adenoviral vectors include one or more adenoviral genes necessary for the processes of replication or packaging of said vector. In addition, the presence of these adenoviral genes entails the risk of possible immunogenic effects when applying in the clinic.

Therefore, this establishes the need for a technique of obtaining high-capacity adenovirus production systems which do not require the use of auxiliary adenoviral vectors and which overcome the drawbacks of the auxiliary vectors known to date.

### SUMMARY OF THE INVENTION

In a first aspect, the invention relates to a polynucleotide (polynucleotide of the invention) or expression vector which comprises a nucleotidic sequence which comprises the genome of a recombinant adenovirus which lacks the ψ packaging sequence and the sequence which encodes the E1A or E1A/E1B protein, wherein said sequence which comprises the genome of a recombinant adenovirus is flanked by specific recognition sequences for a restriction endonuclease.

In another aspect, the invention relates to a host cell which comprises a polynucleotide or expression vector of the invention. In another aspect, the invention relates to a host cell selected from the group consisting of
(i) a host cell which comprises a polynucleotide or expression vector comprising
   (a) a first expression cassette which comprises a nucleotidic sequence which encodes a transcriptional regulator;
   (b) a second expression cassette which comprises a nucleotidic sequence which encodes a restriction endonuclease, and which is under the operational control of a transcriptional regulatory sequence activatable by the transcriptional regulator encoded by the polynucleotide defined in (a);
(ii) a host cell according to (i) which comprises, additionally, a sequence which encodes the adenoviral E1A protein and, optionally, a sequence which encodes the adenoviral E1B protein and
(iii) a host cell which comprises a polynucleotide or expression vector comprising
   (a) a first expression cassette which comprises a nucleotidic sequence which encodes a transcriptional regulator;
   (b) a second expression cassette which comprises a nucleotidic sequence which encodes a restriction endonuclease which is under the operational control of a transcriptional regulatory sequence activatable by the transcriptional regulator (a) and
   (c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a),
wherein said host cell comprises, additionally, a polynucleotide or expression vector of the invention, or which can be obtained by integration in said host cell defined in (i), (ii) or (iii), of a polynucleotide of the invention, wherein the restriction targets which flank the viral genome derived from the polynucleotide or expression vector of the invention are specifically recognized by the endonuclease restriction encoded by said second expression cassette.

In another aspect, the invention relates to a method for the production of cells suitable for the production and packaging of infective particles of a high-capacity adenovirus which comprises
(i) transfecting a cell with
   - a first polynucleotide comprising
      (a) a first expression cassette which comprises a nucleotidic sequence which encodes a transcriptional regulator;
      (b) a second expression cassette which comprises a nucleotidic sequence which encodes a restriction endonuclease, and which is under the operational control of a transcriptional regulatory sequence activatable by the transcriptional regulator encoded by the polynucleotide defined in (a); or
   - a vector which comprises said first polynucleotide, or
   - a second polynucleotide comprising
      (a) a first expression cassette which comprises a nucleotidic sequence which encodes a transcriptional regulator;
      (b) a second expression cassette which comprises a nucleotidic sequence which encodes a restriction endonuclease which is under the operational control of a transcriptional regulatory sequence activatable by the transcriptional regulator (a) and
      (c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a), or
   - a vector which comprises said second polynucleotide and
(ii) transfecting said cell with a polynucleotide of the invention or with a vector which comprises said polynucleotide
wherein stages (i) and (ii) can be carried out in any order or simultaneously and wherein if the cells are transfected in stage (i) with the first polynucleotide or with a vector comprising said first polynucleotide, then cells are used which comprise the sequence which encodes the adenoviral E1A protein and, optionally, the adenoviral E1B protein.

In another aspect, the invention relates to a cell which can be produced by a method in accordance with the invention.

In another aspect, the invention relates to a system for the production of high-capacity adenovirus which comprises
(a) a cell in accordance with the invention and
(b) a high-capacity adenovirus.

In another aspect, the invention relates to a method for the production of high-capacity adenoviral vectors, which comprises the stages of:
(a) transfecting or infecting a host cell with a polynucleotide which comprises the genome of the high-capacity adenovirus which one wants to produce or with an expression vector which comprises said polynucleotide,
(b) cultivating the transfected/infected cells under conditions allowing the replication and packaging ofthe high-capacity adenovirus,
(c) recovering the viral particles of the high-capacity adenovirus and, optionally,
(d) repeating stages (a) to (c) wherein the high-capacity viral particles produced in stage (c) are used in stage (a) of the following cycle,
wherein the host cell used in stage (a) is selected from the group of
(i) a cell which comprises
   - a polynucleotide comprising
      (a) a first expression cassette which comprises a nucleotidic sequence which encodes a transcriptional regulator;
      (b) a second expression cassette which comprises a nucleotidic sequence which encodes a restriction endonuclease which is under the operational control of a transcriptional regulatory sequence activatable by the transcriptional regulator (a)
   - the nucleotidic sequence which encodes the adenoviral E1A protein and, optionally,
   - the adenoviral E1B protein,
   in which case stage (a) is preceded by or carried out simultaneously with a stage of transfection with a polynucleotide of the invention and/or an expression vector which comprises said polynucleotide,
(ii) a cell which comprises a polynucleotide comprising
   (a) a first expression cassette which comprises a nucleotidic sequence which encodes a transcriptional regulator;
   (b) a second expression cassette which comprises a nucleotidic sequence which encodes a restriction endonuclease which is under the operational control of a transcriptional regulatory sequence activatable by the transcriptional regulator (a) and
   (c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a),
   in which case stage (a) is preceded by or carried out simultaneously with a stage of transfection with a polynucleotide of the invention and/or an expression vector which comprises said polynucleotide,
(iii) a cell which comprises a polynucleotide of the invention, in which case stage (a) is preceded by or carried out simultaneously with a stage of transfection with a polynucleotide comprising
   (a) a first expression cassette which comprises a nucleotidic sequence which encodes a transcriptional regulator;
   (b) a second expression cassette which comprises a nucleotidic sequence which encodes a restriction endonuclease, and which is under the operational control of a transcriptional regulatory sequence activatable by the transcriptional regulator encoded by the polynucleotide defined in (a),
   and wherein the cell comprises a sequence which encodes the adenoviral E1A protein and, optionally, the adenoviral E1B protein,
(iv) a cell which comprises a polynucleotide of the invention, in which case stage (a) is preceded by or carried out simultaneously with a stage of transfection with
   - a polynucleotide comprising
      (a) a first expression cassette which comprises a nucleotidic sequence which encodes a transcriptional regulator;
      (b) a second expression cassette which comprises a nucleotidic sequence which encodes a restriction endonuclease which is under the operational control of a transcriptional regulatory sequence activatable by the transcriptional regulator (a) and
      (c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a), and/or
   - an expression vector which comprises said polynucleotide,
(v) a cell which comprises
   - a polynucleotide comprising
      (a) a first expression cassette which comprises a nucleotidic sequence which encodes a transcriptional regulator;
      (b) a second expression cassette which comprises a nucleotidic sequence which encodes a restriction endonuclease, and which is under the operational control of a transcriptional regulatory sequence activatable by the transcriptional regulator encoded by the polynucleotide defined in (a),
   - a polynucleotide of the invention and
   - a sequence which encodes the adenoviral E1A protein and, optionally, the adenoviral E1B protein, and
(vi) a cell which comprises
   - a polynucleotide comprising
      (a) a first expression cassette which comprises a nucleotidic sequence which encodes a transcriptional regulator;
      (b) a second expression cassette which comprises a nucleotidic sequence which encodes a restriction endonuclease which is under the operational control of a transcriptional regulatory sequence activatable by the transcriptional regulator (a) and
      (c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a), and
   - a polynucleotide of the invention.

### BRIEF DESCRIPTION OF THE FIGURES

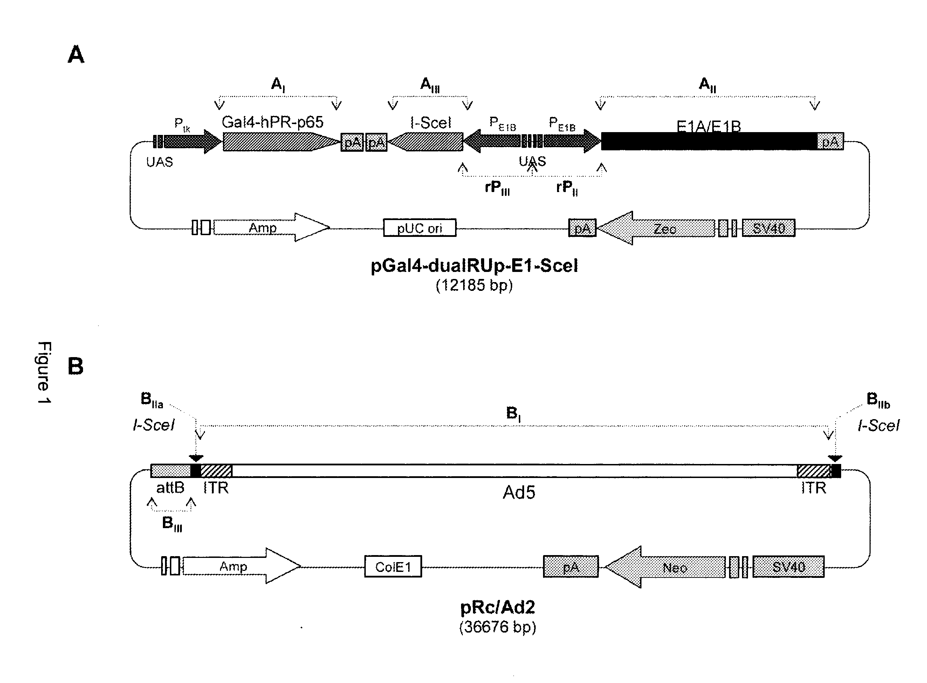

**Figure 1****. A**. Schematic representation of the pGa14-2RU-E1-SceI plasmid which comprises an A2 polynucleotide according to the invention. A_{I}: nucleotidic sequence which encodes a transcription regulator, in this case GAL4-hPR-p65. An: nucleotidic sequence which encodes the adenoviral E1A/E1B proteins. A_{III}: nucleotidic sequence which encodes a restriction endonuclease, in this case I-SceI of *Sacharomyces cerevisiae.* rP_{II} and rP_{III}: promoters which can be regulated by the transcription regulator, GAL4-hPR-p65, which direct the transcription and expression of the E1A/E1B proteins and I-SceI endonuclease respectively. In this embodiment each promoter region comprises a sequence of the TATA box of the adenoviral E1b (P_{E1b}) gene, associated to UAS (*upstream activating sequences*) sequences of GAL4, and where both promoters share the same UAS binding sequences. In this case, the promoter region which directs the transcription and expression of the transcription regulator GAL4-hPR-p65 comprises a minimum promoter of thymidine kinase (Pₜₖ) also associated to UAS (*upstream activating sequences*) of GAL4. Each one of the 3 transcriptional units (GAL4-hPR-p65, E1A/E1B and I-SceI) is completed with a polyadenylation signal (pA). pUCori: origin of replication of the pGeneV5-HisA plasmid used as a base for the construction of the pGa14-2RU-E1-SceI plasmid. Amp: ampicillin resistant gene for the selection of Zeo resistant bacteria. Zeo: Zeocyn antibiotic resistant gene for the selection of resistant clones in eukaryotic cells. SV40: SV40 promoter which directs the expression of the Zeo^{r} resistant gene.
   **B**. Schematic representation of the pRc/Ad2 plasmid which comprises a B polynucleotide according to the invention. B_{I}: nucleotidic sequence which encodes the genome of the adenovirus, in this case serotype 5, an exception of the region which encodes the E1A/E1B proteins and the packaging signal (ψ). The nucleotidic sequence is delimited by ITR (*inverted terminal repeats*) sequences. B_{II}: Specific target sequences for a restriction endonuclease, in this case I-SceI. B_{III}: nucleotidic sequence *attB* which facilitates the integration of the plasmid in the genome of the eukaryotic cell. This process is mediated by the specific and unidirectional PhiC31 recombinase. colE1: origin of replication of the pRC/RSV plasmid used as base for the construction of the pRcAd2 plasmid. Amp: ampicillin resistant gene for the selection of bacteria resistant clones; Neo: Neomycin antibiotic resistant gene for the selection of resistant clones in eukaryotic cells; SV40: SV40 promoter which directs the expression of the Neo^{r} resistant gene pA: polyadenylation signal.

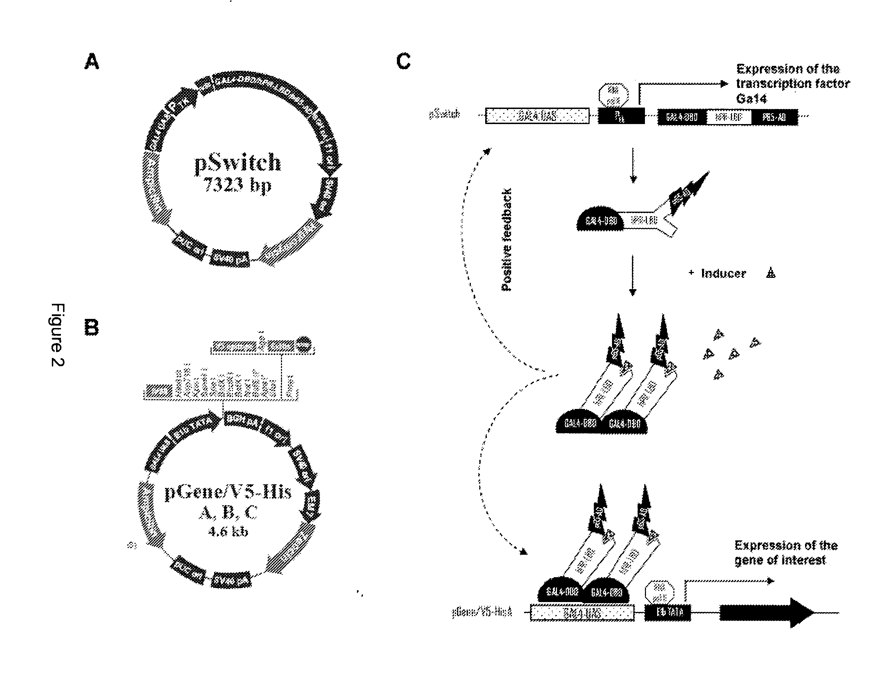

**Figure 2**: Commercial system *GeneSwitch*^{™} (Invitrogen) for the transcriptional control of the gene expression induced by mifepristone.
   A. pSwitch plasmid for expression of the fusion protein composed of the DNA binding domain (GAL4-DBD) of the yeast GAL4 protein, the ligand binding domain of the human progesterone receptor (hPR-LBD) and the activating domain of the human NF-κB protein (p65-AD). The expression of the transcriptional activator GAL4-hPR-p65 is under the control of the minimum promoter of thymidine kinase (Pₜₖ). The binding of the active form of the protein to the GAL4-UAS sequence associated to the promoter regulate its own expression.
   B. pGeneV5-HisA plasmid designed for the cloning of genes of interest. The expression of the cloned gene is under the control of the promoter constituted by the TATA box of E1b (P_{E1b}) and the binding sequences to the transcriptional activator (GAL4-UAS).
   *C. GeneSwitch*^{™} system activation mechanism. The presence of the inducer results in a conformational change of the fusion protein which acts as transcriptional activator and which is expressed constitutively by the pSwitch plasmid. This conformational change consists of the dimerization of the molecule which goes from an inactive state to its active state. The homodimer has the capacity to bind to specific sequences (GAL4-UAS) located in the promoter region of the gene of interest activating its expression (plasmid pGeneV5-HisA) and in the promoter region which controls the expression of the transcription regulator (pSwitch plasmid) starting in this case a positive feedback reaction.

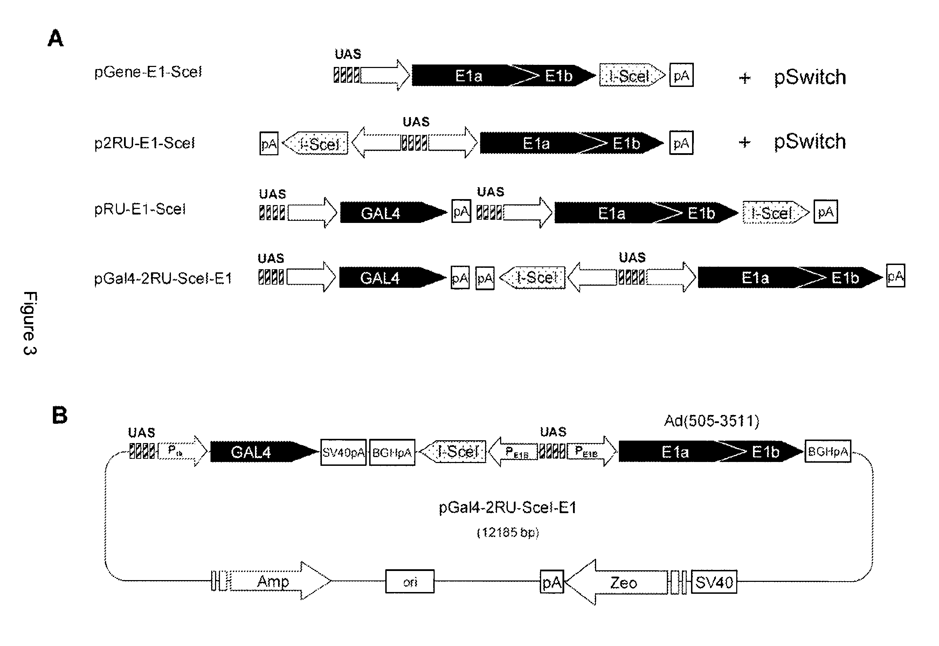

**Figure 3**. Scheme of the regulation systems designed for the transcriptional control of genes E1a, E1b and I-SceI from the *GeneSwitch*^{™} system. The pGene-E1-SceI and p2RU-E1-SceI constructs require the presence of the pSwitch plasmid for the expression of the transcription regulator GAL4-hPR-p65. On the other hand, the constructions pRU-E1-SceI and pGa14-2RU-SceI-E1 include, in a single construction, the genes of E1 region (E1a, E1b), I-SceI gene and the gene which encodes the regulator fusion protein with its specific promoter region. All the genes are under the control of promoters (white arrows) with transcriptional activator binding sequences (UAS). Each one of the transcriptional units is completed with a polyadenylation signal (pA).

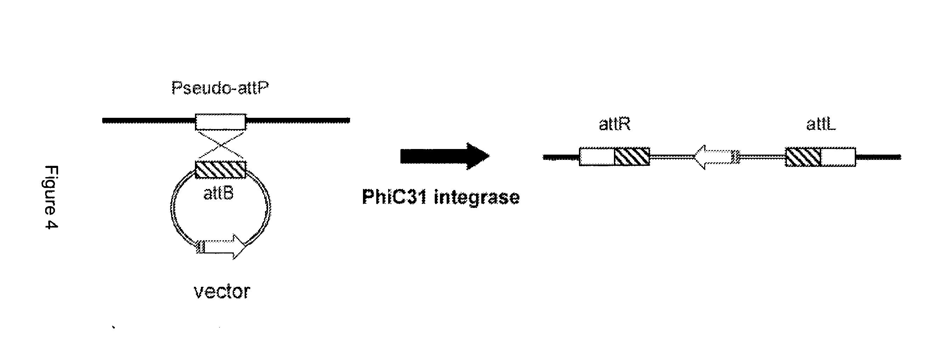

**Figure 4**: Specific integration of exogenous DNA mediated by the PhiC31 integrase of the *Streptomyces* phage. In mammalian cells this integrase is used to promote recombination between the *attB* sequence inserted in the vector with the gene of interest and a pseudo-*attP* sequence present in the cell chromosome. As a result, the original recombination sequences are lost and the integrated vector is flanked by the *attR* and *attL* regions.

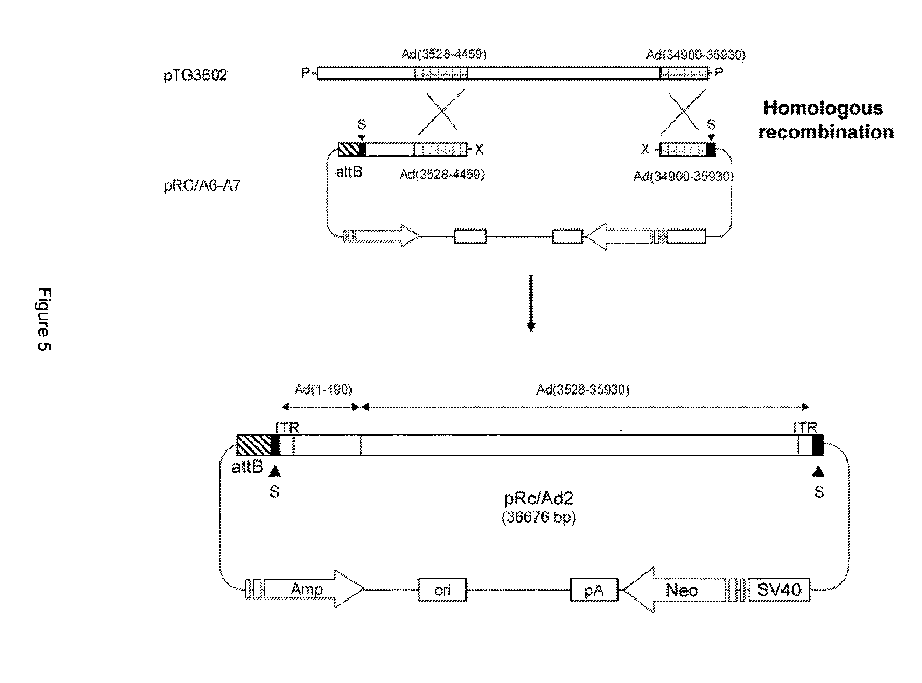

**Figure 5**. Recombination process between homologous sequences of the genome of the serotype 5 adenovirus, purified from the pTG3602 plasmid by digestion with *Pac*I (P), and the pRC/A6-A7 construction, linearized with *Xba*I (X). The numbers indicate the position within the adenoviral genome of each one of the homologous regions. Within the pRC/A6-A7 plasmid the adenoviral sequence is flanked by target sequences for the I-SceI restriction endonuclease (S), whilst at end 5' an *attB* sequence has been inserted for the integration of the plasmid in the mammal cell genome. The pRcAd2 plasmid resulting from the recombination process includes an adenoviral genome deleted between positions 190 and 3528, region corresponding to the encoding sequence of E1a and E1b. ITR: Inverted terminal repeats. colE1: origin of replication of the pRC/RSV plasmid used as base for the construction of the pRcAd2 plasmid. Amp: ampicillin resistant gene for the selection of bacteria resistant clones. Neo: gene resistant to the Neomicyn antibiotic for the selection of resistant clones in eukaryotic cells. SV40: SV40 promoter. pA: polyadenylation signal.

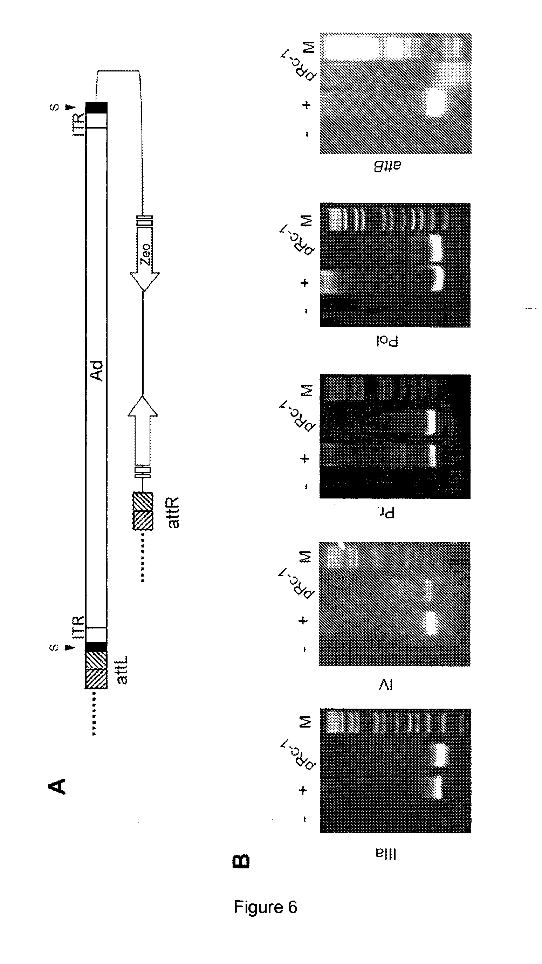

**Figure 6****. A**. Stable integration of the pRcAd2 plasmid in the eukaryotic cell line mediated by PhiC31 recombinase. The insert is flanked by the sequences *attL* and *attR,* product of the recombination between *attB* region of the plasmid and the pseudo-*attP* sequence of the cell genome. S: target for I-SceI endonuclease; Zeo: Zeomycin resistant gene.
   **B**. Detection by Nested-PCR of the adenoviral genes IIIa, IV, protease (Pr) and the gene which encodes the viral polymerase (Pol) from the purified genome DNA of the stable clone pRc-1. The result of the electrophoresis corresponds to the analysis of the amplification reactions with the first internal specifically designed for each gene. The pRc-1 clone was also tested for the detection of the *attB* sequence. The negative result of said amplification confirms the specific integration mediated by the PhiC31 recombinase. (-): negative control of PCR; (+) positive control of PCR; M: molecular weight marker (*1 Kb Plus DNA Ladder;* Invitrogen).

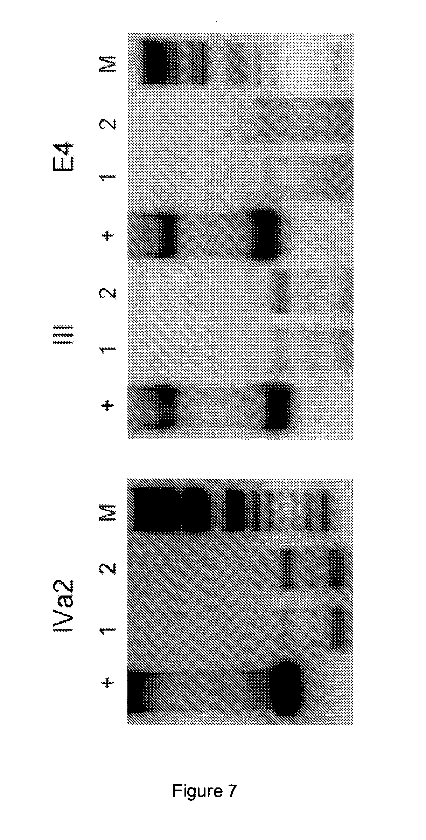

**Figure 7**: RT-PCR of the adenoviral genes IVa2, III and the ORF of 34 Kd of E4 region. The cDNA was obtained from samples of total RNA extracted from cells incubated in the absence (1) and presence (2) of the RU486 inducer. (+): positive control of PCR. M: molecular weight marker (*1 Kb Plus DNA Ladder;* Invitrogen).

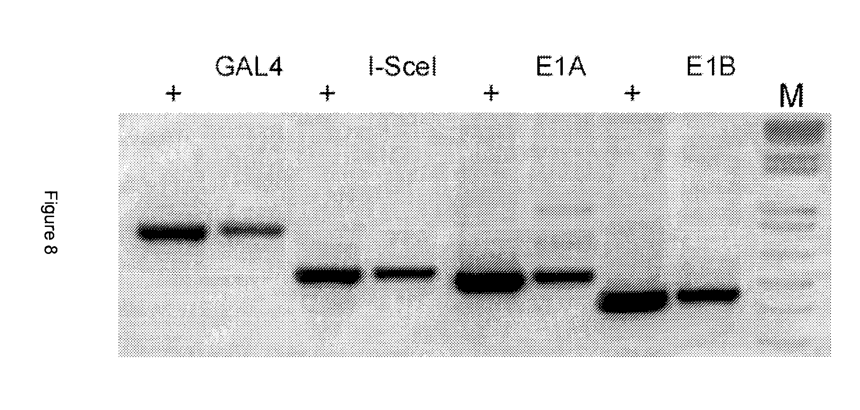

**Figure 8**: Detection of the gene which encodes the transcription regulator GAL4, the I-SceI restriction endonuclease and the genes of the E1 region of the adenovirus (E1a and E1b) by PCR amplification from the genomic DNA of the stable clone Gal-13. (-): negative control of PCR; (+): positive control of PCR; M: molecular weight marker (1 *Kb Plus DNA Ladder;* Invitrogen).

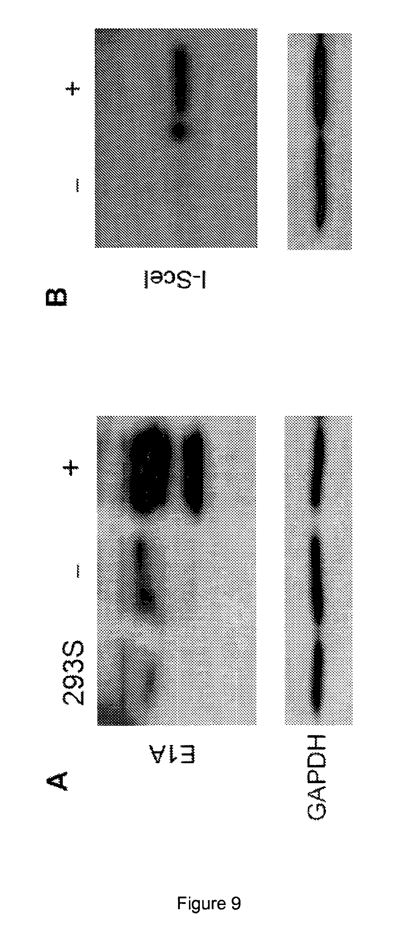

**Figure 9**: Western Blot assay to detect the E1A protein (A) and the I-SceI endonuclease (B). The protein extracts were collected from cells incubated in the absence (-) and presence (+) of the RU486 inducer. The 293S cell line was tested as positive control of the E1A protein GADPH: Positive control of the Western Blot test.

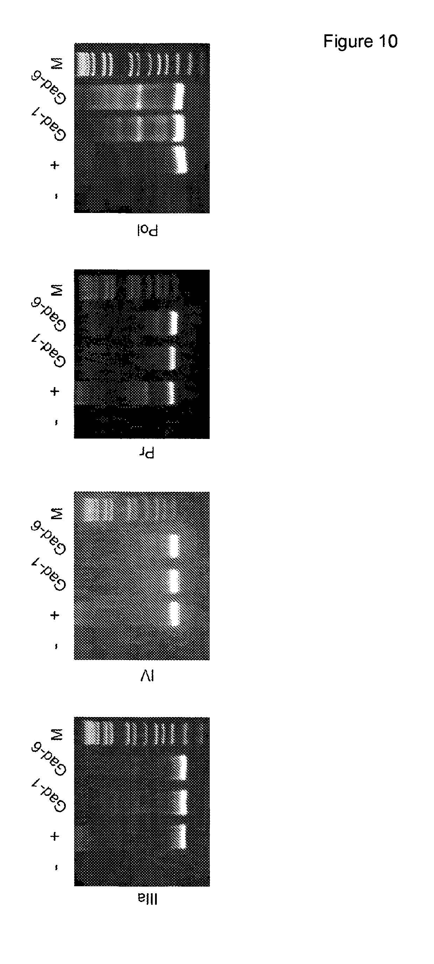

**Figure 10**: Detection by Nested-PCR of the adenoviral IIIa, IV, protease (Pr) genes and of the gene which encodes the viral polymerase (Pol) from the purified genomic DNA of the stable clones GAd-1 and GAd-6. The result of the electrophoresis corresponds to the analysis of the amplification reactions with the internal primers specifically designed for each gene. (-): negative control of PCR; (+) positive control of PCR; M: molecular weight marker (*1 Kb Plus DNA Ladder*; Invitrogen).

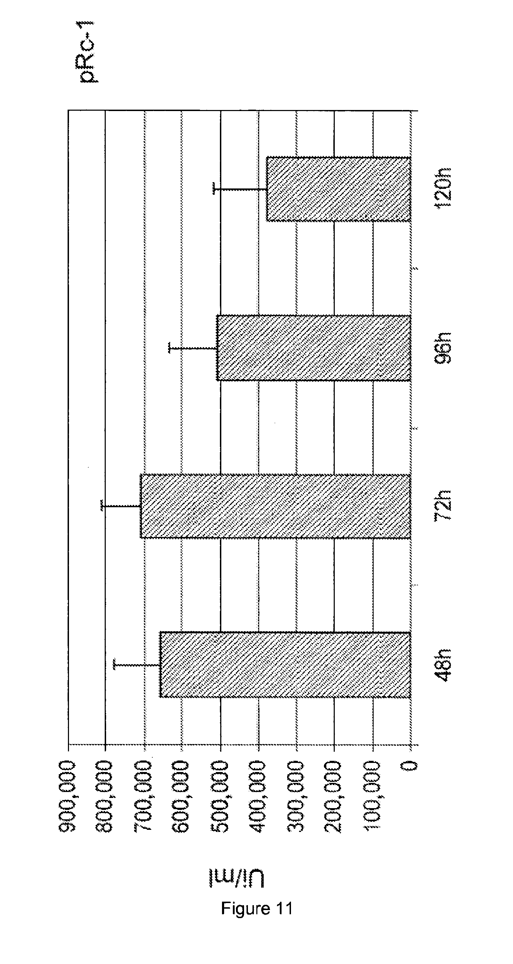

**Figure 11**: Production of KCZ *gutless* adenovirus at passage 0 using the stable clone pRc-1 as packaging line. The virus extracts were collected after 48, 72, 96 and 120 hours, post-transfection, and they were titrated in the 293S cell line. The levels of virus obtained are the average of the three independent experiments and they are expressed in infective units (IU)/ml.

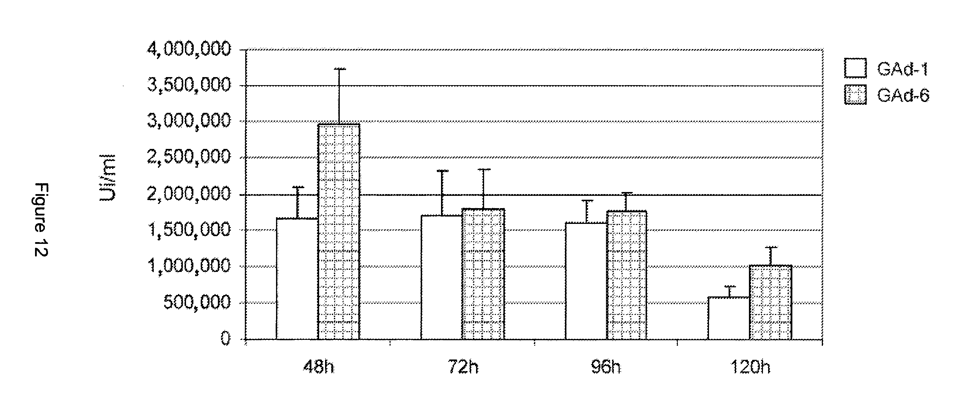

**Figure 12**: Production of KCZ *gutless* adenovirus at passage 0 using as the stable clones GAd-1 and GAd-6 packaging line. The virus extracts were collected after 48, 72, 96 and 120 hours, post-transfection, and they were titrated in the 293S cell line. The levels of virus obtained are the average of the three independent experiments and they are expressed in infective units (IU)/ml.

### DETAILED DESCRIPTION OF THE INVENTION

The authors of the present invention have designed a new production and packaging system (encapsidation) for high-capacity adenoviral vectors which does not require the use of *helper* virus and which is characterized in that it integrates the adenoviral genes necessary for the control, replication and packaging of the viral particles within the genome of a host cell, which improves the stability of the production system in the long term. Likewise, the high-capacity adenoviral vector used only includes the sequences necessary for the replication and packaging of the virions (ITR sequences and ψ signal) so that the cloning capacity of the vector is superior to that shown in other *helper-*independent systems or other auxiliary vectors. Finally, the absence of these adenoviral genes in the high-capacity vector eliminates the risk of any immune response against the vector during its clinical application.

### (Apolynucleotide)

In a first aspect, it is disclosed a polynucleotide (hereinafter A1 polynucleotide of the invention) or an expression vector which comprises:
(a) a first expression cassette which comprises a nucleotidic sequence which encodes a transcriptional regulator;
(b) a second expression cassette which comprises a nucleotidic sequence which encodes a restriction endonuclease, and which is under the operational control of a transcriptional regulatory sequence of the activatable by the transcriptional regulator encoded by the polynucleotide defined in (a).

"Transcription regulator" or "transactivator" are equally understood, in the context of the present invention, as a protein which is capable of activating the transcription of a determined gene by its binding to specific recognition regions for said protein in the non-coding region of said gene. In a particular embodiment, the transactivator or transcription regulator is a regulatable transcription regulator. "Regulatable transcription regulator" is understood, in the context of the present invention, as a transcription regulator whose activity can be modulated by additional factors which can be provided or removed in accordance with the genes which comprise specific binding sites for said regulators. The person skilled in the art will appreciate that the invention includes any method to regulate the expression of the transcription regulator provided that it permits the regulated expression and with a minimum of regulated basal transcription. Thus, it is possible to regulate the transcriptional activator by inducible promoters which respond to an inducer agent, which show a null or negligible basal expression in the absence of inducer agent and which are capable of promoting the activation of the gene localized at position 3'. In accordance with the type of inducer agent, the inducible promoters are classified as Tet on/off promoters (Gossen and Bujard, 1992, *Proc. Natl. Acad Sci. USA,* 89:5547-5551; Gossen *et al.,* 1995, *Science* 268:1766-1769; Rossi and Blau, 1998, *Curr. Opin. Biotechnol.* 9:451-456); Pip on/off promoters (US6287813); antiprogestin-dependent promoters (US2004132086), ecdysone-dependent promoters (Christopherson *et al.,* 1992, *Proc. Natl. Acad. Sci. USA,* 89:6314-6318; No *et al.,* 1996, *Proc. Natl. Acad. Sic. USA,* 93:3346-3351, *Suhr et al.,* 1998, *Proc. Natl. Acad. Sci. USA,* 95:7999-8004; and W09738117), a metallothionein-dependent promoter (WO8604920) and rapamycin-dependent promoters (Rivera *et al.,* 1996, *Nat. Med.* 2:1028-32).

Alternatively, it is disclosed the use of transcriptional regulators whose induction takes place, not by an increase in the expression levels, but by an inducer agent which causes an increase in transcriptional activity, normally, originated by a translocation of the regulator from the cytoplasm to the nucleus. This type of transcriptional regulators are usually formed by a DNA binding domain or DBD, a ligand binding domain or LBD and an activating domain or AD.

The DNA binding domain can be any domain for which there exists a known specific binding element, including synthetic, chimeric or analogous DNA binding domains. Suitable DNA binding domains for the present invention include (i) homeodomains (Scott *et al.,* 1989, *Biochim. Biophys. Acta* 989:25-48; Rosenfeld *et al.,* 1991, *Genes Dev.* 5:897-907) formed, as general rule, by a chain of approximately 61 amino acids which have a secondary structure composed of three alpha helixes, (ii) zinc fingers formed by two to three dozen zinc fingers of DNA of general formula Cys₂His₂ organized in tandem (for example, TFIIIA, Zif268, Gli, and SRE-ZBP) wherein each module comprises an alpha helix capable of contacting with a DNA region of 3 to 5 base pairs, at least 3 zinc fingers being necessary to generate a high-affinity DNA binding site and at least two zinc fingers to generate low-affinity DNA binding sites, (iii) DNA binding domains called helix-turn-helix or HLH such as MAT1, MAT2, MATa1, Antennapedia, Ultrabithorax, Engrailed, Paired, Fushi tarazu, HOX, Unc86, Oct1, Oct2 and Pit-1, (iv) DNA binding domains of the leucine zipper type such as GCN4, C/EBP, c-Fos/c-Jun and JunB. Examples of DNA binding domains suitable in the present invention include the DNA binding domain GAL4, LexA, transcription factors, group H nuclear receptors, nuclear receptors of the steroid/thyroid hormone superfamily. The person skilled in the art will appreciate that the use of hybrid DNA binding domains formed by several DNA binding motifs that may recognized DNA binding sites other than those of the elements comprising them. Thus it is possible to use DNA binding domains formed by the binding of a zinc finger and a *homeobox.* In a preferred embodiment, the DNA binding domain is from the GAL4 protein of *S. cerevisiae.*

The ligand binding sequences capable of promoting the nuclear localization of a transcriptional activator containing them, suitable for use in the present invention, include the localization sequence derived from PPAR (peroxisome proliferator-activated receptor), which are translocated to the nucleus in the presence of 15-desoxy-[Delta]-prostaglandin J2, retinoic acid receptors which are translocated to the nucleus in the presence of alpha, beta or gamma isomers of 9-cis-retinoic acid, farnesoid X receptors, activatable by retinoic acid and TTNPB, hepatic X receptors activatable by 24-hydroxycholesterol, benzoate X receptors, activatable by 4-amino-butylbenzoate, constitutive androstane receptor, pregnan receptors, inducible by pregnolone-16-carbonitrile, steroid and xenobiotic receptors, inducible by rifampycin, progesterone receptors, inducible by medroxyprogesterone as well as by agonists and antagonists of mifepristone and derivatives of 19-nortestosterone, glucocorticosteroid receptors activatable by glucocorticosteroids, thyroid hormone receptors, activatable by T3 and/or T4, and estrogen receptors, activatable by estrogens and their derivatives such as 17-betaestradiol and estradiol, tTA transactivators inducible by "tet-off" tetracyclin/doxyciclin (Gossen and Bujard, 1992, *Proc. Natl. Acad. Sci. USA,* 89: 5547-5551), rtTA transactivators inducible by "tet-on" tetracyclins (Gossen *et al.,* 1995, *Science,* 268: 1766-1769), transactivator inducible by muristerone A or analogue ligands of the ecdysone receptor *(No et al.,* 1996, *Proc. Natl. Acad. Sci. USA,* 93: 3346-3351), transactivators inducible by the RSL1 ligand, such as the RheoSwitch system initially described by Palli *et al.* (2003, *Eur. J. Biochem.,* 270: 1308-1315), a transactivator inducible by rapamycin or analogues of rapamycin *(Ho et al.,* 1996, *Nature,* 382: 822-826; Amara *et al.,* 1997, *Proc. Natl. Acad. Sci. USA,* 94: 10618-10623), and a transactivator inducible by coumermycin/novobiocin, which act competitively as inducer and repressor respectively *(Zhao et al.,* 2003, *Hum. Gene Ther.,* 14: 1619-1629). In a preferred embodiment, the LBD is a ligand binding domain of the human progesterone receptor which can be activated with synthetic antiprogestins including derivatives of progesterone such as RU486 or mifepristone and which do not show any effects in patients as described by *Wang et al., (Proc. Natl. Acad. Sci. USA.* 1994, 91: 8180-4).

Finally, the activating domain can be the activating domain of group H nuclear receptors, nuclear receptors of thyroid or steroidal hormones, the activating domain of VP16, GAL4, NF-κB, B42, BP64, or p65. In a preferred embodiment, the activating domain is that derived from human NF-κB protein.

In a preferred embodiment, the transcriptional activator is the fusion protein called GAL4-hPR-p65 which comprises the DNA binding domain of GAL4 of *Saccharomyces cerevisiae* (Laughon and Gesteland, 1984, *Mol. Cell Biol.,* 4: 260-267; Marmorstein *et al*., 1992, *Nature,* 356: 408-414), the ligand binding domain of human progesterone receptor *(Kastner et al.,* 1990, *Embo J.,* 9: 1603-1614; *Wang et al.,* 1994, *Proc. Natl. Acad. Sci. USA,* 91: 8180-8184) and the activating domain of the human NF-κB protein (Burcin *et al.,* 1999, *Pro. Natl. Acad. Sci. USA,* 96: 355-360; Deloukas and van Loon, 1993, *Hum. Mol. Genet.,* 2: 1895-1900). This transcription regulator is inducible by mifepristone or its analogue RU487. In the absence of mifepristone, the regulator fusion protein is expressed constitutively from the minimum promoter of thymidine kinase. This protein is mainly located in the nucleus and inactive. Upon addition of the inducer mifepristone, or its analogue RU486, this binds with high affinity with the hPR-LBD region of GAL4-hPR-p65, causing a conformational change which results in the dimerization of the protein and its conversion to the active form. These homodimers are capable of binding to specific sites (UAS: *upstream activating sequences)* causing the transcription of genes localized in orientation 3' with respect to said activating sequences and thus activating the transcription of the gene of interest and of the gene which encodes the actual transcription factor (Fig. 2C).

The person skilled in the art will appreciate that this type of transcriptional regulators, whose activity is regulated by the addition of exogenous compounds which promote the translocation of the regulators to the nucleus or their conformational change to active form, do not require that their transcription is subjected to a particularly fine transcriptional control. In general terms, the sequence which encodes the transactivator or transcription regulator is associated to a suitable promoter which directs its transcription and expression, and also to a polyadenylation signal. Since it is not necessary to exactly control the transcription of this type of regulators, they can be found under the control of a constitutive promoter. Without this supposing a limitation, the minimum promoter can be, among others, a constitutive virus promoter such as CMV or RSV, or an eukaryotic cell constitutive promoter, such as PGK or EF1. Alternatively, it is possible to include in the promoter region of the gene which encodes the transcription regulator a response element to the transcription regulator, so that the expression thereof causes an increase in its expression by a positive feedback cycle. In an embodiment, the transcription regulator is regulated by a region formed by one or several binding elements to the GAL4 DNA binding domain, preferably 4 binding elements to the GAL4 DNA binding domain, coupled to the minimum promoter of the thymidine kinase gene (Pₜₖ) of the Herpes Simplex Virus.

The second element of the A1 polynucleotide comprises an expression cassette which encodes a restriction endonuclease which is under the operational control of the transcriptional regulator encoded in the first expression cassette present in the first polynucleotide . The restriction endonuclease is an enzyme which cuts the double stranded DNA by recognition and rupture in specific DNA sites. The endonuclease referred to in the invention is an endonuclease which does not recognize specific sites in the DNA genome of human cells and which, therefore, do not produce ruptures in the native genome of said cells. Without this supposing any limitation, the restriction endonuclease can be, among others, I-SceI, PI-SceI, I-PpoI, I-DmoI or PI-TliI. In a particular embodiment, the restriction endonuclease encoded by the A1 polynucleotide is I-SceI of *Saccharomyces cerevisiae.* The polynucleotide which contains the coding sequence of the restriction endonuclease is under the operational control of a binding sequence to the transcription regulator encoded by the first element of the polynucleotide of the invention. In this way, the promoter responds to the binding of the transactivator activating or repressing the transcription and expression of the endonuclease. However, the capacity of the transactivator for binding and interacting with the promoter is modified depending on whether the regulation factor is bound to a ligand or inducer agent. In consequence, it is possible to regulate the transcription of the gene of interest by the control of the availability of the inducer agent. In some cases the inducer agent acts by activating and favouring the transcription and expression of the gene, whilst in other cases, the presence ofthe inducer agent represses its expression. In the preferred embodiment wherein the transcription regulator is GAL4-hPR-p65, the polynucleotide which encodes the restriction endonuclease is at position 3' with respect to a variable number of binding sequences to the GAL4 DNA binding domain. In an even more preferred embodiment, the gene which encodes the restriction endonuclease is at position 3' with respect to a regulator element composed of 6 binding sequences of the GAL4 transcription factor and the sequence of the TATA box of the E1b (P_{E1b}) adenovirus gene (Lillie and Green, 1989, *Nature,* 338: 39-44).

In a particular embodiment of the A1 polynucleotide, the transcription regulator is GAL4-hPR-p65 and the restriction endonuclease is I-SceI.

### A2 polynucleotide

In a second aspect, it is also disclosed a polynucleotide (hereinafter the A2 polynucleotide) or expression vector which comprises:
(a) a first expression cassette which comprises a nucleotidic sequence which encodes a transcriptional regulator;
(b) a second expression cassette which comprises a nucleotidic sequence which encodes a restriction endonuclease, and which is under the operational control of a transcriptional regulatory sequence activatable by transcription regulator (a) and
(c) a third expression cassette which comprises a nucleotidic sequence which encodes the selected adenoviral E1A protein or the adenoviral E1A and E1B proteins, and which is under the operational control of a transcriptional regulatory sequence activatable by a transcription regulator (a).

Elements (a) and (b) of the A2 polynucleotide essentially correspond to those that form part of the A1 polynucleotide and have been previously described.

Component (c) of the A2 polynucleotide comprises an expression cassette which comprises a nucleotidic sequence which encodes the selected adenoviral E1A protein or the adenoviral E1A and E1B proteins. The genes E1A and, optionally, E1B may come from the adenovirus genome of any.variety or human adenoviral serotype (which have been isolated in humans), for example serotypes 2 (Ad2), 5 (Ad5), 11 (Ad11) or 3 (Ad3). In a particular embodiment, said component includes the sequence delimited between positions 505 and 3511 of the genome of the adenovirus serotype 5 (GenBank: AC000008). This sequence is under the operational control of a regulator sequence of the transcription activatable by transcription regulator (a). In this way, the promoter responds to the binding of the transactivator activating or repressing the transcription and expressing the structural genes E1A and E1B. However, the capacity of the transactivator to bind and interact with the promoter is modified depending on whether the regulation factor is bound to a ligand or inducer agent. In consequence, it is possible to regulate the transcription of the E1A and, optionally the E1B genes by the control of the availability of the inducer agent. In a preferred embodiment, component (c) of the A2 polynucleotide of the invention is under control of a hybrid promoter composed of 6 binding sequences to the GAL4 transcription factor and the TATA box sequence of the E1b (P_{E1b}) adenovirus gene (Lillie and Green, 1989, *Nature,* 338: 39-44).

The person skilled in the art will appreciate that the orientation of the genes in the A2 polynucleotide which respond to the transcriptional activator is not essential for the functioning thereof Thus, in a preferred embodiment, the second and third cassettes are transcribed in opposite direction. In another preferred embodiment, the transcription regulating sequences which control the second and third cassette share the same binding site for the transcriptional regulator (a). In a preferred embodiment, the genes which encode the restriction endonuclease and the adenoviral proteins are under the control of the same hybrid promoter composed of 6 binding sequences to the GAL4 transcription factor and the sequence of the TATA box of the E1b (P_{E1b}) adenovirus gene (Lillie and Green, 1989, *Nature,* 338: 39-44). In an even more preferred embodiment, the second and third cassette of the A2 polynucleotide of the invention are transcribed in opposite directions and share the binding sequences to the DNA binding domain of the GAL4 transcription factor.

In a particular embodiment of the A2 polynucleotide, the first expression cassette (a) comprises the sequence which encodes the transcription regulator GAL4-hPR-p65, the second expression cassette (b) comprises the sequence which encodes the I-SceI restriction endonuclease under the control of one or several binding sites of the GAL4 protein and the third expression cassette (c) comprises the sequences of genes E1A and E1B under the control of one or several binding sites of the GAL4 protein. In an even more preferred embodiment, the regulatable promoters that direct the expression of the sequence which encodes the I-SceI restriction endonuclease and the sequences of genes E1A and E1B share the same response elements for GAL4-hPR-p65.

### Polynucleotide of the invention (B polynucleotide)

In a first aspect, the invention relates to a polynucleotide (hereinafter the B polynucleotide) or expression vector which comprises a nucleotidic sequence which comprises the genome of a recombinant adenovirus and which lacks, at least, the ψ packaging sequence and the sequence which encodes the E1A protein or E1A/E1B protein, wherein said sequence which comprises the genome of a recombinant adenovirus is flanked by specific recognition sequences for a restriction endonuclease. The sequence of the adenoviral genome may be produced from a genome of an adenovirus of any variety or human adenoviral serotype (which has been isolated in humans), for example serotypes 2 (Ad2), 5 (Ad5), 11 (Ad11) or 3 (Ad3).

Preferably, the recognition sequences of the restriction endonuclease are flanked by the adenoviral genome at positions 5' and 3' with respect to the ITR sequences of the ends, where the suitable regions for the replication and transcription of said genome are found. The person skilled in the art will appreciate that the restriction targets used in the B polynucleotide will depend on the restriction endonuclease encoded in the A1 and A2 polynucleotides of the invention. Thus, the target sequences which flank the adenoviral genome are specific recognition sequences for a restriction endonuclease with the same characteristics and requirements indicated previously on describing the endonuclease encoded by the A1 and A2 polynucleotides. Without this supposing any limitation, the sequences may be specific for their recognition, among others, by the I-SceI, PI-SceI, I-PpoI, I-Dmol or PI-TliI endonuclease. In a particular embodiment, they are specific for recognition by the I-SceI endonuclease.

Additionally, in another preferred embodiment, the B polynucleotide in accordance with the invention comprises sequences which facilitate the insertion of said polynucleotide in the genome of the packaging cell. In an even more preferred embodiment, said sequences favouring integration in the genome are sequences of specific recognition for an integrase. Preferably, the target sequence of the integrase is at position 5' with respect to the first recognition sequence for the endonuclease.

Target sequences of integrases suitable for the embodiment of the invention include, without limitation, the target sequence of Cre recombinase of the P1 phage (*LoxP*), the target sequence of FLP recombinase of *S. cerevisiae* (*Frt*), the target sequence of the integrase of the *Streptomyces* ΦC31 phage (*attB, attP*), the target sequence of TP901-1 recombinase, and the target sequence of R4 recombinase, or the target sequence of lambda integrase. In a preferred embodiment, the target sequences of the invention include specific sequences for an integrase of phages which mediate the unidirectional recombination between two specific sites or sequences of the DNA. More particularly, the integrase belongs to the serine integrase family, which is characterized in that they use a catalytic serine residue for the rupture of the DNA strand (Thorpe and Smith, 1998, *Proc. Natl. Acad. Sci. USA,* 95: 5505-5510). The recombination sites recognized by these integrases (*attB* and *attP*) have different sequences and are of shorter than the recombination sequences recognized by other recombinase. The integration mediated by this type of integrase is a unidirectional process strictly controlled by the recombination sequences (Thorpe *et al.,* 2000, *Mol. Microbiol.,* 38: 232-241). Another important characteristic is that the recombination between *attP* and *attB* in the host does not need cofactors to operate. These integrases are currently used in gene manipulation procedures in mammal cells and they have been shown to mediate the efficient integration of genetic material in *attP* sites introduced exogenously or in native sequences which have a partial identity with said *attP* sites (pseudo-*attP*). Without this supposing any limitation, the target sequence of integrase can be a specific recognition sequence for the PhiC31, TP901-1 or R4 integrase, among others. In a particular embodiment, the target sequence of integrase is *att*B, corresponding to the anchoring site in the DNA of bacteria specific for the integrase of the PhiC31 phage. This integrase may mediate the unidirectional recombination between this *attB* sequence and pseudo-*attP* sequences localized in the genome of human cells.

Thus, in another preferred embodiment, the B polynucleotide includes, in direction 5' to 3': optionally, the integrase recognition sequence; the first endonuclease recognition; the adenovirus genome (whose limits are defined by the ITR of the adenovirus); and the second sequence for the restriction endonuclease.

### Vectors and host cells of the invention and methods for their obtainment

The polynucleotides of the invention may be isolated or form part of vectors which permit the propagation of said polynucleotides in suitable host cells. Therefore, in another aspect, the invention relates to a vector, such as an expression vector, which comprises the B polynucleotide of the invention.

The description also discloses a vector, such as an expression vector, which comprises the A1 or A2 polynucleotide.

Suitable vectors for the insertion of the polynucleotides of the invention include expression vectors in prokaryotes such as pUC18, pUC19, Bluescript and their derivatives, mp18, mp19, pBR322, pMB9, CoIE1, pCR1, RP4, phages and "shuttle" vectors such as pSA3 and pAT28, expression vectors in yeast such as vectors of the type of plasmids of 2 microns, integration plasmids, YEP vectors, centromeric plasmids and similar, expression vectors in insect cells such as the vectors of the pAC series and pVL series, expression vectors in plants such as vectors of the series pIBI, pEarleyGate, pAVA, pCAMBIA, pGSA, pGWB, pMDC, pMY, pORE and similar and expression vectors in eukaryotic cells either based on viral vectors (adenovirus, adenovirus-associated virus, as well as retrovirus and, in particular, lentivirus) as well as non-viral vectors such as pSilencer 4.1-CMV (Ambion), pcDNA3, pcDNA3.1/hyg pHCMV/Zeo, pCR3.1, pEFl/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1. Vectors particularly appropriate for the insertion of said polynucleotides are expression vectors in higher eukaryotic cells, either based on viral vectors (such as adeno associated virus, retrovirus, lentivirus, herpesvirus, SV40 and alphavirus) or also based on non-viral vectors (such as plasmids), capable of being maintained and/or replicated and/or expressed in the host cell. In a more particular embodiment, said expression vector is suitable for transfecting or infecting mammal cells *ex vivo,* particularly human cells.

Typically, the eukaryotic expression vectors include, for example, one or more cloned genes and under the transcriptional control of sequences localized at 5' and 3', as well as a selection marker. Without this supposing a limitation, the control sequences include a promoter (including the initial site of the transcription) capable of being expressed in eukaryotic cells, a binding site to ribosomes, a RNA processing signal, a termination site of the transcription and/or a polyadenylation signal.

Preferably, the vectors comprise a reporter or selection gene which gives a phenotype to the cell which expresses it and facilitates the identification and/or selection of those cells which have incorporated the vector after having been put in contact with this. It is preferable that the phenotype of selection is not expressed in the eukaryotic cell prior to its transfection or infection. Useful reporter genes in the context of the present invention include lacZ, luciferase, thymidine kinase, GFP and similar. Useful selection genes in the context of the present invention include, for example, the neomycin-resistant gene, which gives resistance to the G418 aminoglucoside, the hygromycin phosphotransferase gene which gives resistance to hygromycin, the ODC gene, which gives resistance to the ornithine decarboxylase inhibitor (2-(difluoromethyl)-DL-ornithine: DFMO), the dihidrofolate reductase gene which gives resistance to methotrexate, the puromycin N-acetyl transferase gene, which gives resistance to puromycin, the *ble* gene which gives resistance to zeocyn, the adenosine deaminase gene which gives resistance to 9-beta-D-xylofuranoseadenine, the cytosine deaminase gene, which allows the cells to grow in the presence of *N*-(phosphonoacetyl)-L-aspartate, the thymidine kinase gene, which allows the cells to grow in the presence of aminopterin, the Xanthine-guanine phosphoribosyltransferase gene, which allows the cells to grow in the presence of xanthine and in the absence of guanine, the *trpB* gene of *E*. *coli* which allows the cells to grow in the presence of indol instead of tryptophan, the *hisD* gene of *E*. *coli,* which allows the cells to use histidinol instead of histidine. The selection gene is incorporated in a plasmid which may include, additionally, a suitable promoter for the expression of said gene in eukaryotic cells (for example, promoters CMV or SV40), an optimized site of initiation of the translation (for example, a site which follows the so-called rules of Kozak or an IRES), a polyadenylation site such as, for example, the polyadenylation site of SV40 or of the phosphoglycerate kinase, or introns such as, for example, the intron of the beta-globulin gene. Alternatively, it is possible to use a combination of a reporter gene and selection gene simultaneously in the same vector.

The polynucleotides of the invention and the expression vectors that contain them can be produced by conventional techniques of molecular biology and genetic engineering known by any person skilled in the art, and which are recognized in compendiums and manuals such as "Molecular Cloning: a Laboratory manual", by J. Sambrook and D.W. Russel, Eds., Publisher: *Cold Spring Harbor Laboratory Press,* third edition, 2001 (ISBN 978-0879695774). There are also commercial systems which can facilitate the construction of expression vectors for the polynucleotides of the invention. Thus, for example, the construction of an expression vector for mammal cells which includes the A polynucleotide would be facilitated if systems are used such as the *GeneSwitch*™ inducible expression system (Invitrogen, Carlsbad CA92008 USA, Catalog nos. K1060-01 and K1060-2), which permits the construction of a vector for the expression of a gene of interest inducibly by mifepristone; the RheoSwitch™inducible expression system (New England Biolabs, Beverly, MA01915-5599 USA), which permits the expression of a gene of interest inducibly by the ligand RLS I; or any other equivalent commercial system.

The polynucleotides and vectors of the invention may be found forming part of host cells which serve both as propagation vehicle of the vectors and packaging cells for the production of recombinant adenovirus. Thus, in another aspect, the invention relates to a host cell which comprises a B polynucleotide of the invention or an expression vector which comprises any of the aforementioned polynucleotides. If the cells are used as packaging cells for the production of adenovirus, they have to be capable of supporting the production and infection of adenovirus. According to this embodiment, a suitable eukaryotic cell will be any permissive cell for the infection and replication of adenovirus.

In a preferred embodiment, the cell line will be a stable cell line. Without this supposing any limitation, the host cells can be HeLa, A549, KB, HuH7 cells, or any other eukaryotic cell capable of supporting the lifecycle of the adenovirus. If the E1A or E1A/E1B polypeptides are supported by the cell (in the cases wherein the A1 polynucleotide of the invention is used), its expression may be under the control of a regulatable promoter through an inducer agent, for a greater control of the initiation of the expression ofthe adenoviral genes included in the B polynucleotide and thus limit to the maximum the risk of possible cytotoxic effects derived from the excess of viral expression proteins. In this case, a cell will not be suitable which expresses said proteins constitutively, for example a 293 or PerC6 cell.

In a particular embodiment, the host cell is from a mammal, preferably primate and especially human. In a preferred embodiment, the cell line is a lung carcinoma line. In an even more preferred embodiment, the host cells are cells of A549 line. This cell line initiated by Giard *et al.* (1973, *J. Natl. Cancer Inst.,* 51: 1417-1423) and later characterized in depth Lieber *et al.* (1976, *Int. J Cancer.,* 17: 62-70), has a great sensitivity to infection by adenovirus which gives high potential as virus production line (Smith *et al.,* 1986, *J. Clin Microbiol*., 24: 265-268).

In a preferred embodiment, it is dislcosed a host cell which comprises the A1 polynucleotide of the invention or a vector which comprises said polynucleotide. In an even more preferred embodiment, the cell comprises, additionally a sequence which encodes the adenoviral E1A protein and, optionally, a sequence which encodes the adenoviral E1B protein.

In another preferred embodiment, it is disclosed a host cell which comprises the A2 polynucleotide of the invention or a vector which comprises said polynucleotide.

In another embodiment, the invention relates to a host cell which comprises the B polynucleotide of the invention or a vector which comprises said polynucleotide.

The description also discloses a host cell which comprises an A1 or A2 polynucleotide or an expression vector which comprises any of the aforementioned polynucleotides.

In another embodiment, the invention relates to a host cell which comprises the A1 polynucleotide and the B polynucleotide of the invention. In an even more preferred of embodiment, said cell comprises, additionally, a sequence which encodes the adenoviral E1A protein and, optionally, a sequence which encodes the adenoviral E1B protein.

In another embodiment, the invention relates to a host cell which comprises the A2 polynucleotide and the B polynucleotide of the invention.

Both the cells which comprise the A1 and B polynucleotides of the invention and the cells which comprise the A2 and B polynucleotides are particularly suitable for the production and packaging of high-capacity adenoviral vectors for which reason the cells which comprise the A1 and B polynucleotides and the cells which comprise the A2 and B polynucleotides can be used in procedures and systems for the production and packaging of said vectors. However, particularly useful for the production of adenovirus are those wherein the target sites which flank the defective adenoviral genome in the B polynucleotide correspond to the restriction endonuclease encoded by the A1 and A2 polynucleotides, since, otherwise, the restriction endonuclease encoded by the A1 or A2 polynucleotides cannot act on the B polynucleotide releasing the adenoviral genome, which is essential for the replication and transcription of said genome.

In another embodiment, the invention relates to a host cell selected from the group consisting of
(i) a host cell which comprises a polynucleotide or expression vector comprising
   (a) a first expression cassette which comprises a nucleotidic sequence which encodes a transcriptional regulator;
   (b) a second expression cassette which comprises a nucleotidic sequence which encodes a restriction endonuclease, and which is under the operational control of a transcriptional regulatory sequence activatable by the transcriptional regulator encoded by the polynucleotide defined in (a);
(ii) a host cell according to (i) which comprises, additionally, a sequence which encodes the adenoviral E1A protein and, optionally, a sequence which encodes the adenoviral E1B protein and
(iii) a host cell which comprises a polynucleotide or expression vector comprising
   (a) a first expression cassette which comprises a nucleotidic sequence which encodes a transcriptional regulator;
   (b) a second expression cassette which comprises a nucleotidic sequence which encodes a restriction endonuclease which is under the operational control of a transcriptional regulatory sequence activatable by the transcriptional regulator (a) and
   (c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a),
   wherein said host cell comprises, additionally, a polynucleotide or expression vector of the invention, or which can be obtained by integration in said host cell defined in (i), (ii) or (iii), of a polynucleotide of the invention, wherein the restriction targets which flank the viral genome derived from the polynucleotide or expression vector of the invention are specifically recognized by the endonuclease restriction encoded by said second expression cassette.

In a preferred embodiment, the transcriptional regulator encoded by cassette (a) is an activatable transcriptional regulator. In an even more preferred embodiment, the activatable transcriptional regulator (a) is formed by the DNA binding domain of GAL4, the ligand binding domain of the human progesterone receptor and by the activating domain of NF-kappaB p65.

In another preferred embodiment, the restriction endonuclease encoded by cassette (b) is I-SceI.

The host cells of the invention can be obtained either by the introduction of the polynucleotides or vectors of the invention transitorily or, preferably, by the introduction of said polynucleotides of vectors in stable manner. To do this, it is necessary to place in contact the vectors of the invention with the host cells in suitable conditions for the intake of DNA in the cell. The vectors of the invention are introduced in the cells object of study using any of the transfection methods known for the person skilled in the art (see sections 9.1 to 9.5 in Ausubel, F.M. *et al.,* "Current Protocols in Molecular Biology", *John Wiley & Sons Inc;* 2003). In particular, the cells can be transfected by co-precipitation of DNA with calcium phosphate, DEAE-dextran, polybrene, electroporation, microinjection, fusion mediated by liposomes, lipofection, infection by retrovirus and biolistic transfection.

Thus, in another aspect, the invention relates to a method for the production of cell suitable for the production and packaging of infective particles of a high-capacity adenovirus which comprises
(i) transfecting a cell with
   - a first polynucleotide comprising
      (a) a first expression cassette which comprises a nucleotidic sequence which encodes a transcriptional regulator;
      (b) a second expression cassette which comprises a nucleotidic sequence which encodes a restriction endonuclease, and which is under the operational control of a transcriptional regulatory sequence activatable by the transcriptional regulator encoded by the polynucleotide defined in (a); or
   - a vector which comprises said first polynucleotide, or
   - a second polynucleotide comprising
      (a) a first expression cassette which comprises a nucleotidic sequence which encodes a transcriptional regulator;
      (b) a second expression cassette which comprises a nucleotidic sequence which encodes a restriction endonuclease which is under the operational control of a transcriptional regulatory sequence activatable by the transcriptional regulator (a) and
      (c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a), or
   - a vector which comprises said second polynucleotide and
(ii) transfecting said cell with a polynucleotide of the invention or with a vector which comprises said polynucleotide
wherein stages (i) and (ii) can be carried out in any order or simultaneously and wherein if the cells are transfected in stage (i) with the first polynucleotide or with a vector comprising said first polynucleotide, then cells are used which comprise the sequence which encodes the adenoviral E1A protein and, optionally, the adenoviral E1B protein.

The person skilled in the art will appreciate that stages (i) and (ii) in the method described above can be carried out so that the polynucleotides or vectors are in the cell transitorily or, alternatively, can be carried out so that a stable expression of the B polynucleotides is obtained. In the event that it is desired to obtain stable expression of the vectors of the invention in the host cells, it is necessary to include, during the transfection or infection stage, a gene which encodes for resistance to a determined antibiotic, so that it is possible to select those cell lines which have incorporated the DNA in the genome of those cell lines wherein the DNA is in extrachromosomic position. The gene which makes it possible to select the cells can be provided forming part of the same vector which contains the construction object of the invention or, alternatively, it can be separately provided by co-transfection with a second plasmid which contains said resistance gene. In this last case, the plasmid which contains the DNA construction is provided to the transfection mixture in a molar excess with respect to the resistance gene so that for each event of integration of the resistance gene there is a high probability of integration of the gene which contains the promoter object of study. Preferably, the plasmid that contains the DNA construction is provided in an excess of at least 5 times with respect to the vector which contains the reporter gene of resistance. Preferably, the resistance gene is included within the vectors of the invention. The resistance genes suitable for transfection have been previously described. Therefore, in a preferred embodiment, the method of production of cells which comprise the B polynucleotide in accordance with the invention include an additional selection stage wherein cells are selected which have stably integrated the first and/or the second polynucleotide based on selection markers present in the polynucleotides used in stages (i) and (ii).

On the one hand, transfection with an expression vector which comprises the A2 polynucleotide has made it possible to select clones of cells which integrate the polynucleotide in stable manner (maintenance of the cells up to 4 months in culture). Advantageously, it has been verified that the expression of E1A/E1B proteins and of the restriction endonuclease is repressed, maintaining basal expression levels which are not harmful for the cell and that said expression can be induced and activated by the addition to the culture of a suitable quantity of an inducer agent suitable for the specific construction used.

Additionally, in the specific case in which a variant of the B polynucleotide is used which comprises an integrase recognition site, it is necessary to carry out stage (ii) by a co-transfection of the expression vector which comprises the B polynucleotide together with a plasmid which encodes and expresses an integrase and which recognizes the recognition sequence of the integrase, to thus permit the integration of the B polynucleotide in the cell genome. Preferably, in the case that the recognition site of the integrase is the *attB* site, stage (ii) involves a co-transfection with a plasmid which encodes the integrase of the PhiC31 phage, which permits the integration of the B polynucleotide or the vector which contains said polynucleotide in the cell genome by a recombination between the *attB* sequence and pseudo-*attP* sequence present in the chromosomes of human cells. This unidirectional recombination and integration is mediated by the PhiC31 integrase. For the expression of the different viral regions and the subsequent replication of the viral genome present in the B polynucleotide it is necessary that the ITR ends of the virus are free, in addition to also requiring the presence of the viral E1A/E1B proteins. Consequently, the cells generated carry the genome of the defective adenovirus but this cannot be expressed or replicated. Although it is possible that integrations also occur of the B polynucleotide in random manner, the integration in pseudo-*attP* sequences is majority and without losses of genetic material.

In a preferred embodiment, the transcriptional regulator encoded by cassette (a) is an activatable transcriptional regulator. In an even more preferred embodiment, the activatable transcriptional regulator (a) is formed by the DNA binding domain of GAL4, the ligand binding domain of the human progesterone receptor and by the activating domain of NF-kappaB p65.

In another preferred embodiment, the restriction endonuclease encoded by cassette (b) is I-SceI.

The methods previously described permit the production of cell lines suitable for the packaging of high-capacity adenoviral vectors. Thus, in another aspect, the invention relates to cells obtained by any of the methods previously described. In particular, the invention contemplates cells which comprise the polynucleotide , wherein said polynucleotide can be integrated stably in the cell genome or can be found transitorily.

### Systems and methods for the production and packaging of adenoviral vectors of the invention

The packaging cells produced by the introduction of the polynucleotides of the invention or of the vectors that comprise them in cell lines can be used for the generation of high-capacity adenoviral vectors by the introduction in said cells of adenoviral genomes which comprise the gene of interest, either by transfection with polynucleotides which comprise the adenoviral genome or by infection with recombinant adenoviruses which comprise said gene. After the infection/transfection with the adenovirus/polynucleotide which comprises the adenoviral genome which comprises, in turn, the gene of interest, and the placing in contact of the cells with the agent which promotes the expression of the genes included in the A1 or A2 polynucleotide, the transcription regulator starts to be produced in the cell which, in turn, activates the transcription of the gene which encodes the restriction endonuclease. This, in turn, acts in the target sites which flank the deficient adenoviral genome comprised in the B polynucleotide, causing its excision from the cell genome, which is a necessary condition for the subsequent transcription of the adenoviral genes that form part of said genome. The transcription of the adenoviral genes from the B polynucleotide also require the expression of the early E1A and E1B genes which are encoded either by the A2 polynucleotide, in which case they respond to the activation of the transcription regulator, or in the actual packaging cell, also under the control of the transcription regulator.

Thus, in another aspect, the invention relates to a system for the production of high-capacity adenovirus which comprises
(a) a cell in accordance with the invention and
(b) a high-capacity adenovirus

In another aspect, the invention relates to a method for the production and packaging of high-capacity adenoviral vectors, which comprises stages of:
(a) transfecting or infecting a host cell with a polynucleotide which comprises the genome of the high-capacity adenovirus which production is intended or with an expression vector which comprises said polynucleotide,
(b) cultivating the transfected/infected cells in conditions which permit the replication and packaging of the high-capacity adenovirus,
(c) recovering the viral particles of the high-capacity adenovirus and, optionally,
(d) repeating stages (a) to (c) wherein the high-capacity viral particles produced in stage (c) are used in stage (a) of the following cycle,
wherein the host cell used in stage (a) is selected from the group of,
(i) a cell which comprises
   - a polynucleotide comprising
      (a) a first expression cassette which comprises a nucleotidic sequence which encodes a transcriptional regulator;
      (b) a second expression cassette which comprises a nucleotidic sequence which encodes a restriction endonuclease which is under the operational control of a transcriptional regulatory sequence activatable by the transcriptional regulator (a)
   - the nucleotidic sequence which encodes the adenoviral E1A protein and, optionally,
   - the adenoviral E1B protein,
   in which case stage (a) is preceded by or carried out simultaneously with a stage of transfection with a polynucleotide of the invention and/or an expression vector which comprises said polynucleotide,
(ii) a cell which comprises a polynucleotide comprising
   (a) a first expression cassette which comprises a nucleotidic sequence which encodes a transcriptional regulator;
   (b) a second expression cassette which comprises a nucleotidic sequence which encodes a restriction endonuclease which is under the operational control of a transcriptional regulatory sequence activatable by the transcriptional regulator (a) and
   (c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a),
   in which case stage (a) is preceded by or carried out simultaneously with a stage of transfection with a polynucleotide of the invention and/or an expression vector which comprises said polynucleotide,
(iii) a cell which comprises a polynucleotide of the invention, in which case stage (a) is preceded by or carried out simultaneously with a stage of transfection with a polynucleotide comprising
   (a) a first expression cassette which comprises a nucleotidic sequence which encodes a transcriptional regulator;
   (b) a second expression cassette which comprises a nucleotidic sequence which encodes a restriction endonuclease, and which is under the operational control of a transcriptional regulatory sequence activatable by the transcriptional regulator encoded by the polynucleotide defined in (a),
      and wherein the cell comprises a sequence which encodes the adenoviral E1A protein and, optionally, the adenoviral E I B protein,
(iv) a cell which comprises a polynucleotide of the invention, in which case stage (a) is preceded by or carried out simultaneously with a stage of transfection with
   - a polynucleotide comprising
      (a) a first expression cassette which comprises a nucleotidic sequence which encodes a transcriptional regulator;
      (b) a second expression cassette which comprises a nucleotidic sequence which encodes a restriction endonuclease which is under the operational control of a transcriptional regulatory sequence activatable by the transcriptional regulator (a) and
      (c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a), and/or
   - an expression vector which comprises said polynucleotide,
(v) a cell which comprises
   - a polynucleotide comprising
      (a) a first expression cassette which comprises a nucleotidic sequence which encodes a transcriptional regulator;
      (b) a second expression cassette which comprises a nucleotidic sequence which encodes a restriction endonuclease, and which is under the operational control of a transcriptional regulatory sequence activatable by the transcriptional regulator encoded by the polynucleotide defined in (a),
   - a polynucleotide of the invention and
   - a sequence which encodes the adenoviral E1A protein and, optionally, the adenoviral E1B protein, and
(vi) a cell which comprises
   - a polynucleotide comprising
      (a) a first expression cassette which comprises a nucleotidic sequence which encodes a transcriptional regulator;
      (b) a second expression cassette which comprises a nucleotidic sequence which encodes a restriction endonuclease which is under the operational control of a transcriptional regulatory sequence activatable by the transcriptional regulator (a) and
      (c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a), and
   - a polynucleotide of the invention.

Stage (a) of the method for the production and packaging of high-capacity adenoviral vectors comprises the putting in contact of a packaging cell with a high-capacity adenovirus or with a polynucleotide or vector which comprises the genome of said adenovirus.

"High-capacity adenovirus" is understood, according to the invention, as recombinant adenoviruses of those called auxiliary-dependent or *gutless,* characterized in that they lack all the genes of the adenoviral genome except regions ITR 5' and 3' and the ψ packaging region, as described, for example, by Alba *et al.* (2005, *Gene Therapy,* 12:S18-S27). These adenovirus admit heterologous sequences of up to 36 kb since they lack their own adenoviral genome sequences. Heterologous genes that may be incorporated in the *gutless* vectors in accordance with the methods of the invention include the genes which encode erythropoietin (EPO), leptins, corticotropin-releasing hormone (CRH), growth hormone releasing hormone (GHRH), gonadotropin-releasing hormone (GnRH), thyrotropin-releasing hormone (TRH), prolactin releasing hormone (PRH), melatonin releasing hormone (MRH), Prolactin-releasing hormone (PIH), somatostatin, adrenocorticotropic hormone (ACTH), somatropic or growth hormone (GH), luteinizing hormone (LH), follicle-stimulating hormone (FSH), thyrotropin (TSH or thyroid stimulating hormone), prolactin, oxytocin, anti-diuretic hormone (ADH or vasopressin), melatonin, Müllerian inhibitor factor, calcitonin, parathyroid hormone, gastrin, cholecystokinin (CCK), secretin, PBGD, insulin-like growth factor I (IGF-I), insulin-like growth factor II (IGF-II), Atrial natriuretic peptide (APN), Human chorionic gonadotropin (hCG), insulin, glucagon, somatostatin, pancreatic polypeptide (PP), leptin, neuropeptide Y, rennin, angiotensin I, angiotensin II, factor VIII, factor IX, tissue factorr, factor VII, factor X, thrombin, factor V, factor XI, factor XIII, interleukin 1 (IL-1), interleukin 2 (IL-2), tumour necrosis factor alpha (TNF-α), interleukin 6 (IL-6), interleukin 8 (IL-8 and chemokins), interleukin 12 (IL-12), interleukin 16 (IL-16), interleukin 15 (IL-15), interleukin 24 (IL-24), alpha, beta, gamma interferons, CD3, ICAM-1, LFA-1, LFA-3, chemokins including RANTES 1α MIP-1α, MIP-1β, neuronal growth factor (NGF), platelet-derived growth factor (PDGF), Transforming growth factor beta (TGF-beta), bone morphogenetic proteins (BMPs), fibroblast growth factors (FGF and KGF), epidermal growth factor (EGF and related), vascular endothelial growth factor (VEGF), granulocyte-colony stimulating factor (G-CSF), glial cell derived neurotrophic factor, (GDNF), keratocyte growth factor, endothelial growth factor, antitrypsin 1 alpha, tumor necrosing factor, granulocyte and macrophage colony stimulating factor (GM-CSF), cardiotrophin 1 (CT-1), oncostatin M (OSM), anfiregulin (AR), cyclosporin, fibrinogen, lactoferrin, tissue plasminogen activator (tPA), chymotrypsin, immunoglobulins, hirudin, superoxide dimutase, imiglucerase, β-Glucocerebrosidase, glucosidase-α, α-L-iduronidase, iduronate-2-sulfatase, galsulfase, α-human galactosidase A, proteinase inhibitor α-1, lactase, pancreatic enzymes (lipase, amylase, protease), adenosine deaminase, immunoglobulin, albumin, botulinum toxins of type A and B, collagenase, human deoxyribonuclease I, hyaluronidase, papain, L-asparaginase, lepirudin, streptokinase, transforming growth factor beta peptides (TGF-β) such as those described in WO0331155 WO200519244 and WO0393293, whose content is incorporated in the present invention by reference, suitable expression cassettes for the transcription of interference RNA molecules (shRNA, siRNA, miRNA, modified RNA of ribonucleoproteins U1). In addition to said heterologous genes, the *gutless* adenovirus used in the invention may contain stuffer regions, such as the stuffer sequences derived from the genome of the lambda phage described by Parks *et al.* (1999, *J*. *Virol.,* 73:8027-8034) or the stuffer DNA which appears in pSTK120 as described by Sandig *et al.* (2000, *Proc. Natl. Acad. Sci. USA,* 97:1002-1007). These regions typically contain sequences present in the human genome and are capable of increasing the expression of the heterologous genes with respect to the adenovirus controls which, instead of said human stuffer DNA comprise the 22 kb fragment of the lambda phage described in Parks *et al.* (1999, *J*. *Virol., 73:8027-8034).* The total length of the human stuffer DNA may vary but oscillates between 20 and 30 kb, preferably from 20 to 25 kb or, even more preferably, from 21 to 23 kb, for example 22 kb. The number of sequences that act in *cis* in said stuffer DNA may vary between 2, 3, 4 or more and include, without limitation, the repeated alphoid domain of 16.2 kb (described in Smith *et al.,* 1995, *Mol. Cell. Biol.,* 15:5165-5172); matrix adhesion regions (MAR) such as, for example, a 4.2 kb fragment which contains the left end of the type 6 adenovirus or two copies of the matrix adhesion region of immunoglobulin κ (as described by Betz *et al.,* 1994, *Cell,* 77:239-248); a hepatocyte control region (HCR) such as the 1.2 kb fragment which comprises said HCR (as described by Miao *et al.,* 2000, *Molecular Therapy,* 1:522-532); or another matrix adhesion region such as the region of 2.8 kb of the chicken lysozyme (as described by Phi-Van *et al.,* 1990, *Mol. Cell. Biol.* 10:2302-2307, matrix adhesion region of region 5' of interferon β (as described by Bode *et al.* 1992, *Science,* 10:195-197) or other non-coding human sequences such as telomeric or centrometic sequences.

The putting in contact of the host cells with the polynucleotide which comprises the adenoviral genome is carried out using techniques known by the person skilled in the art, such as transfection of said polynucleotide or of a vector which comprises it by co-precipitation of DNA with calcium phosphate, DEAE-dextran, polybrene, electroporation, microinjection, liposome mediated fusion, infection by retrovirus and biolistic transfection. Alternatively, it is possible to put in contact the cells with the adenovirus that one wants to amplify, in which case the actual capacity of the adenovirus to infect cells permits the introduction in the cell of said genome.

"Packaging cell" is understood as any of the cells described in the present invention and which contain, at least, the A1/A2 polynucleotide or the B polynucleotide of the invention. The person skilled in the art will appreciate that it is possible to use different packaging cells. However, depending on the presence or not of one or more of the polynucleotides of the invention in the packaging cell stage (a) should be preceded by or be carried out simultaneously with a transfection stage wherein the cells are put in contact with those polynucleotides of the invention which do not appear in the packaging cells, and which are necessary to provide the elements essential for the replication of the adenoviral genome as well as for the production in the cells of the polypeptides necessary for the assembly of the adenovirus. Thus, it is possible to use as packaging cell a cell which comprises the A1 polynucleotide and a sequence which encodes adenoviral E1A protein and, optionally, adenoviral E1B protein. However, the use of this packaging cell requires a stage previous to or simultaneous with stage (a) consisting of the transfection of said cell with the B polynucleotide of the invention or an expression vector which comprises said polynucleotide.

In the case that a cell is used which comprises the A2 polynucleotide stage (a) should be preceded by or carried out simultaneously with a stage of transfection with the B polynucleotide of the invention or with an expression vector which comprises said polynucleotide.

In the case that a cell comprising the B polynucleotide of the invention is used as packaging cell, stage (a) should be preceded by or carried out simultaneously with a stage of transfection with the A1 polynucleotide and, in which case, the cell should comprise, additionally, a sequence which encodes the adenoviral E1A protein and, optionally, the adenoviral E1B protein.

In the case that a cell is used which comprises the B polynucleotide of the invention, stage (a) should be preceded by or carried out simultaneously with a stage of transfection of the A2 polynucleotide or with an expression vector which comprises said polynucleotide.

It is also possible to use a cell which comprises the A1 polynucleotide , the B polynucleotide of the invention and a sequence which encodes adenoviral E1A protein and, optionally, adenoviral E1B protein or, alternatively, a cell which comprises A2 polynucleotide and B polynucleotide of the invention.

Stage (b) of the method of the invention comprises maintaining the transfected/infected cells obtained in stage (a) in conditions which permit the replication and packaging of high-capacity adenovirus. For this, it is necessary that said conditions permit the expression of the transcription regulator encoded by the A1 or A2 polynucleotide, so that this acts on their binding sites in the regulator region of the gene of the endonuclease present in the A1 and A2 polynucleotide and on its binding sites in the regulator region of the adenoviral E1A and E1B genes, present in the A2 polynucleotide. To do this, in the cases wherein the transcription regulator encoded by the A1 and A2 polynucleotide is an activatable regulator, stage (b) involves putting in contact cells with the agent capable of activating the transcription regulator. In a preferred embodiment, the transcription regulator encoded by the A1 and A2 polynucleotide is GAL4-hPR-p65, as previously described, in which case, the inducer agent is mifepristone.

In a preferred embodiment, the transcriptional regulator encoded by cassette (a) is an activatable transcriptional regulator. In an even more preferred embodiment, the activatable transcriptional regulator (a) is formed by the DNA binding domain of GAL4, the ligand binding domain of the human progesterone receptor and by the activating domain of NF-kappaB p65.

In another preferred embodiment, the restriction endonuclease encoded by cassette (b) is I-SceI.

Stage (b) is carried out during the time necessary so that the concentration of viral particles is sufficiently high, as determined by standard means such as the HPLC-based Resource Q method, as describd by Shabram *et al.,* (1997, *Hunt. Gene Ther.,* 8:453-465), or indirectly by visual inspection of the cytopathic effect in the cells. Optionally, the cell culture may include the addition of fresh medium.

In stage (c), the adenoviruses generated during stage (b) are recovered from the cell culture. To do this, it is necessary to separate the cells of the culture medium by any method known to a person skilled in the art (trypsinization and centrifugation or filtration), lysing the cells in inert solution (cycles of freezing/thawing, homogenization, sonication, cavitation, use of detergents and similar) and purifying the adenovirus particles by known methods such as ultracentrifugation in CsCl gradient or by different types of chromatography such as combination of ionic chromatography and metal chelate affinity chromatography *(Huyghe et al.,* 1996, *Hun. Gene Ther.,* 6:1403-1416), a combination of ion exchange chromatography and gel fractioning chromatography (WO07059473), chromatography in diethylaminoethyl (DEAE) cellulose (Haruna *et al.,* 1961, *Virology* 13, 264-267), chromatography in hydroxyapatite (US2002/0064860) and chromatography in other resins such as soft gels (agarose), macroporous polymers based on synthetic polymers which include permission chromatography (fractogel) and materials based on a soft gel in a hard capsule (for example, Ceramic Hyper® F) (Rodrigues, 1997, *J. Chromatogr. B Biomed. Sci. Appl.,* 699:47-61).

Stage (d), which is optional, consists of repeating stages (a) to (c) one or several times. To do this, in each stage (a) of the cycle, the adenoviral particles obtained in stage (c) of the previous cycle are used as starting material. In this way, the process of the invention can be repeated several times obtaining a greater number of adenoviral particles in each cycle.

The invention is illustrated below based on the following examples provided by way of illustration and non-limitative of the scope of the invention.

### EXAMPLES

### Example 1: Construction of the pGal4-2RU-E1-SceI plasmid for the inducible expression of the E1a and E1b adenoviral genes and of the I-SceI restriction enzyme.

In the present embodiment, the pGa14-2RU-E1-SceI plasmid, which contains the A2 polynucleotide as defined in the invention (Fig. 1A), has been specifically designed for the control, on a transcriptional level, of the expression of the adenoviral genes E1a and E1b. Within this construction, and under the same type of transcriptional control, the gene encoding the I-SceI restriction endonuclease has been included. The transcription regulator which controls the expression of genes E1a, E1b and I-SceI, is constitutively expressed from a nucleotidic sequence included within the A2 polynucleotide.

The mechanism of regulation of transcription, included in the present invention, is mediated by the antiprogestin system mifepristone *(Burcin et al.,* 1999, *Proc. Natl. Acad. Sci. USA,* 96: 355-360). Said regulation takes place via a fusion protein insensitive to endogenous human hormones but active in the presence of synthetic antiprogestins (RU486) at sufficiently low doses to avoid secondary effects in humans.

Based on the construction of the pGa14-2RU-E1-SceI plasmid, the commercial system *GeneSwitch*^{™} (Invitrogen, Carlsbad CA92008 USA, Catalog nos. K1060-01 and K1060-2) has been used. This system provides all the genetic components necessary for the design of the plasmid which includes the A2 polynucleotide.

The first component of the *GeneSwitch*^{™} system is the pSwitch vector (Fig. 2A), which encodes the fusion GAL4-hPR-p65 protein which acts as transcriptional activator. The protein is divided in a DNA binding domain (DBD) of the GAL4 protein of *Saccharomyces cerevisiae* (Laughon and Gesteland, 1984, *Mol. Cell Biol.,* 4: 260-267; Marmorstein, *et al.,* 1992, *Nature,* 356: 408-414), a truncated ligand-binding domain of the human progesterone receptor (hPR-LBD) (Kastner, *et al.,* 1990, *Embo J.,* 9: 1603-1614; *Wang, et al.,* 1994, *Proc*. *Natl. Acad. Sci. USA,* 91: 8180-8184) and the activating domain of the human NF-κB protein (AD) *(Burcin, et al.,* 1999, *Proc. Natl. Acad. Sci. USA,* 96: 355-360; Deloukas and van Loon, 1993, *Hum. Mol. Genet.,* 2: 1895-1900). The expression of the regulator protein is under the control of a hybrid promoter derived from the binding of activation sequences of GAL4 of *Saccharomyces cerevisiae* (UAS: *upstream activating sequences)* (Giniger *et al.,* 1985, *Cell,* 40: 767-774; Wang *et al.,* 1994, *Proc. Natl. Acad. Sci. USA,* 91: 8180-8184) and the minimum promoter of the gene thymidine kinase (Pₜₖ) of the Herpes Simplex Virus. The activation region of the promoter includes 4 copies of the sequence 5'- (T/C) GGAGTACTGTCCTCCG-3' (SEQ ID NO:1). Each one of them serves as binding site for two molecules of the DNA binding domain of the GAL4 transcription factor (Marmorstein, *et al.,* 1992, *Nature,* 356: 408-414).

The second component of the *GeneSwitch*^{™} system is the pGene/V5-HisA plasmid (Fig. 2B), which controls the expression of the gene of interest. In this case the hybrid promoter which controls the transcription is composed of 6 sequences of binding of the GAL4 transcription factor and the TATA box sequence of the E1b (P_{E1b}) adenovirus gene (Lillie and Green, 1989, *Nature,* 338: 39-44).

In the absence of mifepristone, the system will constitutively express the regulator fusion GAL4-hPR-p65 protein from the thymidine kinase minimum promoter. This protein is predominantly localized in the nucleus and inactive. At the time of addition of the inducer mifepristone, or its analogue RU486, this binds with high affinity to the hPR-LBD region of GAL4-hPR-p65, causing a conformational change which results in the dimerization of the protein and its conversion to the active form. These homodimers interact with binding sequences of GAL4 (UAS) localized in the pGene/V5-HisA and pSwitch plasmids, thus activating the transcription of the gene of interest and the gene which encodes the actual transcription factor (Fig. 2C).

Based on the idea suggested in the present invention of placing the E1 region of Ad5 and the gene of I-SceI endonuclease under the control of the inducible system *GeneSwitch*™, different combinations of the regulation system, of the sequence of the adenoviral genome between positions 505 and 3511 (Ad5(505-3511)) (GenBank: AC000008) and of the coding gene of I-SceI were initially designed. In these combinations, the regulation mechanism is based on the use of one or two plasmids and wherein genes E1 and gene I*-Sce*I remain under the control of a single promoter or under the control of different promoters arranged, in turn, in the same or different orientation (Fig. 3A). Each one of these combinations is tested to determine their regulatory capacity of the expression of E1 region and gene I*-Sce*I and its stable integration capacity within the cell genome. From all these, the pGa14-2RU-E1-SceI construction was considered the most suitable for its application in the design of an independent high-capacity adenovirus production system of *helper.*

The bacterial strains used for the propagation of the constructions described bellow were *Escherichia coli* DH5a and *E. coli* STBL2. The growth is carried out in Luria Bertani medium (LB) (Sigma-Aldrich, 28760 Madrid, Spain) supplemented with ampicillin (100 µg/ml) (Sigma-Aldrich) as selection medium and at 37°C, in the case of the DH5α strain, and 30 °C for STBL2. The bacteria were transformed following the CaCl₂ protocol initially described by Hanahan (1983, *J. Mol. Biol.,* 166: 557-580). To do this, 10-20 ng of plasmid DNA were mixed with 100 µl of competent cells and it was kept in ice for 30 min. Then, in order to increase the efficiency of the process, the mixture was incubated at 42 °C for 1 minute and it was immediately transferred to ice wherein it was incubated for a further 2 min. Finally, 1 ml of LB medium were added and it was incubated for 1 h at 37°C and under stirring.

The purification of plasmids and the purification of the DNA fragments resulting from the digestion with restriction enzymes after their separation in agarose gels was carried out, respectively, using the FX *microplasmid prep kit* systems (GE Healthcare Europe, 8290 Barcelona, Spain) and QI*Aquick Gel extraction kit* (Qiagen, Valencia CA91355, USA) following the manufacturer's recommendations.

In the cloning of DNA fragments, the ligation reactions were designed for a final volume of 20 µl with 1 U of T4-DNA ligase (New England Biolabs, Beverly MA01915-5599, USA) and 2 µl of 10X reaction buffer (New England Biolabs). In the case of fragments with blunt ends, the reactions were designed for a final volume of 20 µl with 1 U of T4-DNA ligase (New England Biolabs), 2 µl 10X reaction buffer (New England Biolabs) and 2 µl of 50% PEG 4000. In all cases, all the reactions were incubated at ambient temperature for 2 hours and they were inactivated at 65 °C/10 minutes before their introduction in the corresponding E. *coli* strain. The recombinant clones were selected based on the resistance marker encoded by the vector and they were confirmed by digestion with restriction endonucleases and electrophoresis in 1% agarose gel.

All the PCR reactions designed in the synthesis process of the pGa14-2RU-E1-SceI plasmid were carried out in the presence of 1 U of the *Taq* polymerase enzyme (*BioTaq,* Bioline, London NW2 6EW, UK), PCR buffer (Bioline), MgCl₂ 4 mM, 0.4 mM of each dNTP, 10 pmol of each primer, DNA and sterile deionized water until a final volume of 50 µl. The amplification conditions were the following: 1 denaturing cycle of 4 minutes at 95 °C; 29 cycles of 50 seconds of denaturing at 94 °C, 40 seconds of hybridization with the primer at 50°C and 40 seconds of elongation at 72 °C; 1 x 5 minute cycle at 72 °C of final elongation. For the analysis of the amplification products, the samples were mixed with 1.5 µl of the loading solution (0.25% bromophenol blue in distilled water; 40% Glycerol; 100 mM EDTA) and they were analysed by electrophoresis in agarose gels (1%) with TAE 1X buffer (40 mM Tris Base; 20 mM glacial acetic acid; 1 mM EDTA, pH 8.0) displaying the DNA by staining with ethydium bromide (0.5 µg/ml).

The pGa14-2RU-E1-SceI regulation plasmid was designed in five steps as described below:

### 1) Construction of the pGene-LinkerRU2 vector:

The pSwitch plasmid was linearized with the *Sbf*I (position 38) and *BamH*I (position 2484) restriction enzymes to include the LinkerRU2. This was previously synthesized from the primers
LinkerRU-1: LinkerRU-2:

In the annealing reaction, 10 µl of each primer was mixed at a 50 mM concentration in buffer solution (50 mM Tris-HCl pH8.0; 100 mM NaCl; 1 mM EDTA). The reaction conditions were the following: 95°C for 2 minutes and 52 °C for 10 minutes. Next the LinkerRU2 primer was phosphorylated with the T4 enzyme kinase polynucleotide following the manufacturer's recommendations (New England Biolabs). The reaction was incubated for 30 minutes at 37°C and it was inactivated at 65 °C for 15 minutes. Then, the ligation occurred of LinkerRU2 with the pSwitch vector and it was transformed in *E*. *coli* DH5α. The resulting construction was called pSwitch-LinkerRU2.

Next, the digestion of the pSwitch-LinkerRU2 vector with the *FspI* enzymes (position 6623 of pSwitch) and *Apa*I (position 2721 of pSwitch) released LinkerRU2 dragging part of the adjacent sequences of the pSwitch vector. This fragment was subcloned in the pGeneV5-HisA vector previously digested with these same enzymes. The resulting vector is called pGene-LinkerRU2 and will be the base of the construction of the pGal4-2RU-E1-SceI vector.

### 2) Cloning of the E1 region of the adenovirus:

From the pGL3-Basic vector (Groskreutz and Schenbom, 1997, *Methods Mol. Biol.,* 63: 11-30) and by digestion with *BamH*I and *Xba*I*,* a fragment of 262 pb was released which included the polyadenylation signal of SV40 (SV40pA). This sequence was cloned in the pBluescript II KS(+) plasmid (Stratagene, La Jolla CA92037, USA), previously digested with these same enzymes, to give rise to the pBS-SV40pA plasmid.

In parelell, the sequential digestion of the pGeneV5-HisA plasmid with the *SalI* (position 151) and *Pvu*II (position 895) enzymes permitted the isolation of the polyadenylation signal of the bovine growth hormone (BGHpA). This fragment of 744 pb was cloned in the pBS-SV40pA plasmid previously linearized with *Sal*I and *BamH*I and whose *BamH*I end was previously made blunt with *Klenow* following the manufacturer's recommendations (*DNA Polymerase I, Large (Klenow) fragment;* New England Biolabs). In this way, an insert was produced which comprises the polyadenylation signals BGH and SV40 joined by a target sequence for the *BamH*I restriction enzyme.

Next, the pGeneLinkerRU2-2pA plasmid was synthesized. To do this, the digestion of the pBS-BGHpA-SV40pA vector with *Xba*I released a fragment of 623 pb which includes the two polyadenylation sequences and which was cloned within the unique *Xba*I site present in the LinkerRU2 of the pGene-LinkerRU2 plasmid. The correct orientation of the insert (5'-SV40pA-BGHpA-3') was confirmed by restricting with *Pme*I.

The E 1 region of the Ad5 was amplified by PCR with the primers:
ad19 5'-GAGTGCCAGCGAGTAGAGTT-3' (SEQ ID NO: 4), and
ad22 5'-GAATTCTCAATCTGTATCTTCATC-3' (SEQ ID NO: 5).

The resulting product of 3 kb was cloned in the specific vector for PCR pGemT-Easy products (Promega Biotech Ibérica, 28108 Madrid, Spain). By digestion with *Eco*RI said E1 region was purified and it was subcloned within the *Eco*RI target of the pGeneLinkerRU2-2pA vector and it was located "downstream" from the BGHpA polyadenylation signal. The resulting plasmid was called pGeneLinkerRU2-2pA-E1.

### 3) Cloning of the GAL4-DBD/hPR-LBD/p65-AD gene (transcription regulator) and its self-regulating promoter region.

The digestion of the pSwitch vector with the SbfI (position 38) and *BamH*I (position 2485) restriction enzymes isolated the binding sequences to the transcription regulator (UAS region), the minimum promoter of the kinase protein (Ptk) and the gene encoding the transcriptional activator GAL4-DBD/hPR-LBD/p65-AD. This *Sbf*I*-BamH*Ig fragment of 2.4 kb, was cloned in the pGeneLinkerRU2-2pA plasmid previously digested with the same enzymes and causing the loss of the polyadenylation sequence SV40pA. The resulting construction received the name of pGeneLinkerRU2-Gal4-BGHpA-E1.

To recover this second polyadenylation signal, the SV40pA sequence was purified from the initial pGeneLinkerRU2-2pA construction by digestion with *BamH*I*.* The fragment of 0.2 kb was cloned within the sole target *BamH*I of the pGeneLinkerRU2-Gal4-BGHpA-E1 plasmid.

### 4) Cloning of the I-SceI gene

The gene which encodes the I-SceI endonuclease was obtained by PCR with the primers:
SceI-b1 5'-AAACCATGGGATCAAGATCGC-3' (SEQ ID NO:6), and
SceI-b2 5'-CCCTCTAGATTATTTCAGGAAAGTTTCGGAGGA-3' (SEQ ID NO:7),
from the pI-SceI plasmid (Choulika, *et al.,* 1994, *C*. *R. Acad. Sci. III,* 317: 1013-1019). After its cloning in pGemT-Easy it was released by digestion with the *Not*I restriction enzyme. The ends of the purified fragment were made blunt with the *Klenow* fragment following the manufacturer's recommendations (New England Biolabs) and then it was subjected to a second digestion with the *Spe*I restriction enzyme. Finally, the purified fragment, of 0.8 kb, was cloned between the *Spe*I (position 478) and *Not*I (position 522, treated with *Klenow*) targets of the pGeneV5-HisA vector. Treatment with *Klenow* entails the loss of the *Not*I target. The resulting plasmid was called pGene-ISceI.

The digestion of the pGene-ISceI plasmid with *Xba*I released a fragment of 1.2 kb which includes the gene of endonuclease I-SceI under the transcriptional control of the minimum promoter of the E1b adenovirus gene (P_{E1B}). The fragment was subcloned within the localized *Spe*I compatible target, within the pGeneLinkerRU2-Gal4-2pA-E1 plasmid, between the polyadenylation sequence BGH and the E1 region. The resulting construction was called pGeneLinkerRU2-Gal4-2pA-ISceI-E1.

### 5) Cloning of the dual inducible promoter for the control of the transcription of I-SceI and the E1 region of the adenovirus.

The region of the pGeneV5-HisA vector which includes the regulator sequences of binding to the transcriptional activator (UAS) and the minimum promoter P_{E1B} was obtained by amplification by PCR with the primers:
PRU-1 5'-AATACTAGTCCGAGCTCTTACGCGGGTC-3' (SEQ ID NO:8), and
PRU-2 5'-AATACGCGTGGTGGCCTGTGAAGAGAAAAAA- 3' (SEQ ID NO:9),
and it was subcloned in the pGemT-Easy vector. Later, the insert was released by digestion with *Not*I and it was subcloned in the GeneLinkerRU2-Gal4-2pA-ISceI-E1 construction, within the *Not*I target localized between the minimum promoter P_{E1B} of 1-SceI and the E1 region of the adenovirus. The resulting construction was called pGal4-2RU-SceI-E1 (Fig. 3B).

### Example 2: Construction of the pRCAd2 plasmid for the integration in mammal cells of the genome of an adenovirus deleted in the packaging signal and genes E1a and E1b, and which encodes the I-SceI endonuclease.

Within the sequence that includes the adenoviral genome, the packaging sequence (ψ) and the region corresponding to the early genes E1a and E1b were deleted in order to achieve a greater control of the expression of the other viral genes. Likewise, the adenoviral sequence is delimited at both ends by recognition sequences for the I-SceI restriction endonuclease of *Saccharomyces cerevisiae* (Anglana and Bacchetti, 1999, *Nucleic Acids Res.,* 27: 4276-4281) which, due to the size of their target sequence (TAGGGATAACAGGGTAA - SEQ ID NO:8) and their high specificity, does not recognize sequences within the cell genome to transfect. The insertion of these sequences facilitates the release of the viral genome only in the presence of the I-SceI protein. This digestion leaves free the ITR ends of the adenovirus, necessary for the correct transcription and replication of the viral DNA.

Finally, the 5' end of the construction has included an *attB* sequence which promotes the integration of the plasmid within the cell genome, a process mediated by the PhiC31 recombinase of the *Streptomyces* phage. This enzyme has a great potential as integration system of exogenous DNA in mammal cells since it gives rise to a specific and unidirectional reaction and possesses net frequencies of integration higher than those observed with other recombinases (Thyagarajan *et al.,* 2001, *Mol. Cell. Biol.,* 21: 3926-3934). The recognition sequences for PhiC31 integrase are called *attB* and *attP* and are different in their sequence. After the integration reaction, the resulting *att* sequences differ from the original *attB* and *attP* sequences, blocking possible secondary recombination reactions. In human cells, PhiC31 is capable of mediating recombination events at points of the genome where there exist sequences with partial identity at *attP* and which are called pseudo-*attP* (Fig. 4). According to Thyagarajan *et al.* (2001, *supra*) the total number of pseudo-*attP* sequences could be between 10² and 10³ which makes the integration frequency 2 to 3 times greater than in the case of integrations mediated by homologous recombination, and even 10 to 100 times greater than the integration mediated by other specific recombinases such as the Cre recombinase.

The strains of *E. coli* used for the propagation of the constructs described below were DH5α, STBL2 and BJ5183. The bacteria were transformed following the CaCl₂ protocol described in Example 1. The growth took place in Luria Bertani medium (LB) (Sigma-Aldrich) supplemented with Ampicillin (100 µg/ml) (Sigma-Aldrich) as selection medium and at 37°C, in the case of the DH5α and BJ5183 strains, and 30°C for *E. coli* STBL2.

All the PCR reactions designed in the synthesis process of the pRcAd2 plasmid were carried out in the same conditions as those described in Example 1. The general procedures applied in the purification and manipulation of nucleic acids is also described in Example 1.

The pRcAd2 vector was designed in 4 steps, described below:

### 1) Cloning of the attB-ISceI-Ad(1-190) sequence

The first step in the construction of the pRcAd2 vector was the amplification by PCR of the *attB* region with the primers:
attB-P1 5'-AGATCTGTCGACGATGTAGGTCACGGTCTCCGAAGC-3' (SEQ I D NO: 11) and
attB-P2 5'-TTAATTAATTACCCTGTTATCCCTAGTCGACATGCCCGCCGTGA-3' (SEQ ID NO: 12),
using as template the pTA-attB plasmid *(Groth, et al.,* 2000, *Proc. Natl. Acad. Sci. USA,* 97: 5995-6000). The amplified fragment was subcloned in the specific vector for PCR pGemT-Easy products (Promega), following the manufacturer's recommendations. The attB-P2 primer includes a linker with the recognition sequence for the I-SceI restriction enzyme of *Saccharomyces cerevisiae.*

The *attB*-I-SceI fragment (316 pb) was released from the pGemT-Easy vector by digestion with the restriction enzymes ***Nco*I** and *Pac*I and it was subcloned in the pGemT/A6 plasmid, previously digested with these same enzymes. pGemT/A6 derives from the amplification by PCR of the region of the adenovirus genome serotype 5 included between positions 1 and 190, with the primers:
ad63: 5'-AGATCTAGGGATAACAGGGTAATCATCATCAATAATATACCTT-3', (SEQ ID NO: 13) and
ad64: 5'-AGATCTCGGCGCACACCAAAAACGTC-3' (SEQ ID NO:14),
and its cloning in the pGemT-Easy vector.

In the resulting construction, pGemT/attB-A6, the restriction target for **I-SceI** was inserted between the *attB* region and position 1 of the adenovirus genome.

### 2) Cloning of the Ad(3528-4459) region

The insert attB-ISceI-Ad(1-190), of 512 pb, was excised by digestion with *Bgl*II and it was subcloned in the pRC/A4-A5 vector. This vector is a product of the amplification of the adenovirus sequence included between positions 3528 and 4459 with the primers:
ad57 5'-AGATCTCGTGGCTTAAGGGTGGGAAA-3' (SEQ IDNO:15), and
ad58: 5'-TCTAGATTATATGGCTGGGAACATAGCC-3' (SEQ ID NO:16).
and its cloning with the eukaryote expression pRC/RSV (Invitrogen), which further includes the ampicillin resistant gene, the neomycin resistant gene for its selection in mammal cells. For the insertion of the Ad(3528-4459) sequence in pRC/RSV, *Bgl*II and *Xba*I target sequences were included at the 5' end of the primers ad57 and ad58, respectively. The insert was integrated between positions 13 and 701 of the vector.

The construct resulting from the ligation of the *attB-Sce*I*-*Ad(1-190) fragment to vector pRC/A4-A5 was called pRC/A6-A5. In this way, E1 region of the adenovirus was deleted and it would be localized between positions 190 and 3528.

### 3) Cloning of the Ad(34901-35938) region

The 3' end of the adenoviral genome (positions 34901-35938) was amplified with the primers:
ad59: 5'-TCTAGATTAGCTATGCTAACCAGCGTAG-3'(SEQ ID NO:17), and
ad65: 5'-GGTACCTAGGGATAACAGGGTAATCATCATCAATAATATACCTT-3'(SEQ ID NO:18).

The target sequence was included at end 5' of the ad65 primer for the I-SceI restriction enzyme (underlined). Both primers include in their 5' the restriction targets for *Xba*I (ad59) and *Kpn*I (ad65). The fragment of 1060 pb, product of this amplification, was cut with these enzymes to facilitate their "downstream" subcloning of the Ad(3528-4459) region inserted in the pRC/A6-A5 vector. The resulting construction was called pRC/A6-A7.

### 4) Obtainment of the pRcAd2 plasmid

The pRC/A6-A7 plasmid was linearized with *Xba*I and dephosphorylated with Alkaline Phosphatase following the manufacturer's recommendations (*Alkaline Phosphatase, Calf Intestinal (CIP);* New England Biolabs).

Simultaneously, the digestion of the pTG3602 plasmid (Chartier, *et al.,* 1996, *J. Virol.,* 70: 4805-4810) with *Pac*I released a complete copy of the genome of the adenovirus serotype 5. Its recirculation was avoided by the dephosphylation of its ends with alkaline phosphatase.

Both the linearized vector pRC/A6-A7 and the released insert of the pTG3602 plasmid were used simultaneously to transfer the electrocompetent bacterial strain of *E. coli* BJ5183 (*BJ5183 Electroporation competent cells;* Stratagene). These cells are especially designed to promote recombinant processes between homologous sequences of a transferring vector, which contains the gene of interest, and a vector which includes the adenoviral genome. In this case the homologous regions included in both DNA fragments (Ad(3528-4459) and Ad(34901-35938 regions) favours said process of recombination whose resolution entails the insertion of the adenoviral genome inside the pRC/A6-A7 vector and after position 4459 of the virus (Fig. 5).

The electroporation process was carried out, following the manufacturer's recommendations, in a *Gene Pulser* (Biorad) electroporation system and establishing the following parameters: voltage: 2.5 kV; resistance: 200 Ω; capacity: 25 µF; pulse duration: 5 m. Finally, 1 ml of LB medium was added and the suspension was incubated for 1 hour at 37 °C. Next serial dilutions of the transformed cells were prepared. They were seeded in plates of culture medium supplemented with ampicillin (100 µg/ml) as selection antibiotic for the pRC/A6-A7 plasmid. The incubation was continued at 37°C until the appearance of resistant clones.

The restriction analysis with *Hind*III confirmed the correct insertion of the adenoviral genome in only one of the clones obtained and which was called pRcAd2.

The pRcAd2 plasmid includes the complete genome of the adenovirus, with the exception of the region localized between positions 190 and 3528 and which corresponds to the E 1 region. The sequence is delimited by restriction targets for I-SceI, and has an *attB* sequence at its end 5' (Fig. 5).

### Example 3: Construction and characterization of stable cell lines for the production and packaging of high-capacity adenoviral vectors

The present invention proposes the production of stable cell lines designed for the production and packaging of high-capacity adenoviral vectors. These cells will be capable of producing all the components necessary for the replication, assembly and functioning of the virus.

Due to the cytotoxicity caused by elevated levels of viral proteins, a transcriptional regulation system inducible by mifepristone controls the expression of genes E1a and E1b, which also directs the expression and replication of the rest of the adenoviral genome. This same system controls, in turn, the production of the I-SceI restriction endonuclease necessary for the release of the adenoviral genome integrated in the genome of the eukaryotic cell, which provides a second level of transcription control. The induction (and therefore the production of viral particles) starts at the time of addition to the culture medium of mifepristone or its analogue RU486.

As it is not necessary to use a *helper* virus during the production process, the system developed by the inventors permits the production of viral extracts without the undesirable residual contamination of viral particles which contain genomic copies of said *helper* virus.

In the present embodiment the human cell line of lung carcinoma A549 was used for the design of a production system of independent high-capacity *helper* vectors. This cell line initiated by Giard *et al.* (1973, *J. Natl. Cancer Inst.,* 51: 1417-1423) and later characterized in depth by Lieber *et al.* (1976, *Int. J. Cancer.,* 17: 62-70), has great sensitivity to infection by adenovirus which gives a high potential as virus production line (Smith *et al.,* 1986, *J*. *Clin. Microbiol.,* 24: 265-268). The cells can easily be cultured without variations in their morphology and remain viable between 10 and 14 days without showing important signals of degeneration.

### Example 3.1: Generation of cell lines by transfection of the pRcAd2 plasmid for the stable integration of the adenoviral genome (B polynucleotide).

To produce a packaging cell line capable of complementing the synthesis of viral proteins involved in the replication, formation of the caspsid and packaging of high-capacity adenovirus, the A549 cells were incubated in a 10 cm² plate at 37°C, 5% CO₂, until a confluence of 80% was reached. The cells were co-transfected by lipofectamine with 3 µg of the pRcAd2 construction (gives resistance to Neomycin (neo^{r})) and 1 µg of the pCMV-Int plasmid (Groth *et al.,* 2000, *Proc. Natl. Acad. Sci. USA,* 97: 5995-6000), which encodes the integrase of the PhiC31 phage of *Streptomyces* and which promotes the stable integration of the plasmid in the cell genome (Fig. 6A).

The DNA of both plasmids was previously diluted in 500 µl of medium without *OPTI-MEM* serum (Gibco^{®}: Invitrogen, Carlsbad CA92008, USA). In order to enhance the formation of the complexes between the DNA and cationic lipids, the reagent *Plus Reagent* (Invitrogen) was added in a 1:0.75 ratio (DNA: *Plus Reagent).* After 5 minutes of incubation at ambient temperature 6 µl of *Lipofectamine LTX* (Invitrogen) was added and the mixture was incubated again at ambient temperature for 25 minutes. Next, the dilution was added drop by drop on the cell culture medium: DMEM supplemented with 10% foetal bovine serum (Gibco^{®}); 1% L-Glutamin (200 mM in 0.85% NaCl; Lonza Ibérica, 08006 Barcelona, Spain); and 1% Penicillin/Streptomycin (10 mg/ml in 0.85% NaCl; Gibco^{®}). After 4 hours of incubation at 37 °C, 5% CO₂, the medium was replaced by fresh DMEM medium and the incubation continued after another 24 hours. Finally, the medium was removed and, after washing with 1X saline phosphate buffer (PBS) (Gibco^{®}), the transfected cells were raised with trypsin (*Trypsin-EDTA;* Gibco^{®}) and they were centrifuged at 1250 rpm for 5 minutes at ambient temperature. The cell precipitate was resuspended in 6 ml of culture medium and they were distributed in three 60 cm² plates. Fresh medium was added, in this case supplemented with the selection antibiotic G418, analogue of Neomycin, at a concentration of 600 µg/ml. Every two days, the culture medium was changed until the appearance of isolated clones resistant to antibiotic (2-3 weeks). The clones were raised with trypsin and they were transferred to 0.3 cm² wells with selection medium. The incubation was continued until 80% confluence was reached and then they were transferred to wells of greater surface area. After successive steps a clone resistant to G418 was isolated which received the name of pRc-1. To prove the stability of the clone, the cells were kept for 4 months under culture without any changes in their morphology or viability being observed.

In order to confirm the integration of the pRcAd2 plasmid in the isolated clone, genomic DNA of the pRc-1 clone was extracted with the *QIAmp DNA minikit* (Qiagen) system following the manufacturer's recommendations. This DNA was used in Nested-PCR assays to detect the presence of the adenoviral genes IIIa, IV, the gene encoding protease and the gene which encodes the viral polymerase. The primers used in this detection were:

| Gene | Primer | Sequence | Tm PCR | SEQ ID NO: |
|---|---|---|---|---|
| IIIa | IIIa1 | 5'-AGAGCCAGCCGTCCGGCCTTA-3 | 55°C | 19 |
| | IIIa2 | 5'-CGCGGTCGCCTGTGGGAGCCC-3 | | 20 |
| | IIIa3 | 5'-GGTTGCACTAAACGCCTTCC-3' | 54°C | 21 |
| | IIIa4 | 5'-AGCCCCTGCAAGTTTTTGAA-3' | | 22 |
| | IV1 | 5'-CCTCTAGTTACCTCCAATGGC-3' | 45°C | 23 |
| IV | IV2 | 5'-AGTAACTTTAGTTTGCAAGGA- 3' | | 24 |
| | IV3 | 5'-GGGCAACACACTCACCATGC-3' | 55°C | 25 |
| | IV4 | 5'-ACATGCAAAGGAGCCCCGTA-3' | | 26 |
| | Pr1 | 5'-ACAGCTGCCGCCATGGGCTCC-3' | 55°C | 27 |
| Protease | Pr2 | 5'-GGGCGAGTGGCGCTCCAGGAA-3' | | 28 |
| | Pr3 | 5'-GCTTTTCTGACCAGCGACT-3' | 51°C | 29 |
| | Pr4 | 5'-CACTGTGGCTGCGGAAGTAG-3' | | 30 |
| | POL1 | 5'-CTGGCCTGGACGCGAGCCTTT-3' | 55°C | 31 |
| Polymerase | POL2 | 5'-CAGGGTCCTCGTCAGCGTAGT-3' | | 32 |
| | POL3 | 5'-TACGGGGACACGGACAGCCTT-3' | 60°C | 33 |
| | POL4 | 5'-CTTGGCGCGCAGCTTGCCCTT-3' | | 34 |

The PCR reactions with the external primers of Nested-PCR (primers 1 and 2) were carried out in the presence of 1 U of the enzyme *Taq* polymerase (*BioTaq,* Bioline), PCR buffer (Bioline), 1.5 mM MgCl₂, 0.4 mM of each dNTP, 10 pmol of each primer, DNA and sterile deionized water until a final volume of 50 µl. The amplification conditions were the following: 1 cycle of 2 minutes at 94°C, 50 seconds at 94 °C of denaturing, 50 seconds of hybridization with the primer (Tm according to the gene to be amplified, see table) and 50 seconds at 72°C of elongation; 35 cycles of 50 seconds at 94 °C of denaturing, 50 seconds of hybridization with the primer (Tm according to the gene to be amplified, see table) and 50 seconds at 72°C of elongation; 1 cycle of 7 minutes at 72 °C of final elongation. The amplification reactions with the internal primers of Nested-PCR (primers 3 and 4) were carried out from 2 µl of the previous PCR reactions and in the presence of 1 U of the enzyme *Taq* polymerase (*BioTaq,* Bioline), PCR buffer (Bioline), MgCl₂ 2.5 mM, 0.4 mM of each dNTP, 10 pmol of each primer, DNA and sterile deionized water until a final volume of 50 µl. In this case the amplification conditions were: 35 cycles of 50 seconds at 94 °C of denaturing, 50 seconds of hybridization with the primer (Tm according to the gene to be amplified, see table) and 50 seconds at 72 °C of elongation; 1 cycle of 7 minutes at 72 °C of final elongation.

The loss of the *attB* region was also verified by PCR reaction using primers attB-P1; attB-P2 (Example 2). The PCR reactions were carried out in the presence of 1 U of the enzyme *Taq* polymerase (*BioTaq,* Bioline), PCR buffer (Bioline), 4 mM MgCl₂, 0.4 mM of each dNTP, 10 pmol of each primer, DNA and sterile deionized water until a final volume of 50 µl. The amplification conditions were the following: 1 cycle of denaturing of 4 minutes at 95 °C; 29 cycles of 50 seconds at 94 °C of denaturing, 40 seconds at 50 °C of hybridization with the first and 40 seconds at 72 °C of elongation; 1 cycle of 5 minutes at 72 °C of final elongation.

To analyse the amplification products, the samples were mixed with 1.5 µl of the loading solution (0.25 % bromophenol blue in distilled water; 40% Glycerol; 100 mM EDTA) and they were analysed by electrophoresis in agarose gels (1%) with TAE 1X buffer (40 mM Tris Base; 20 mM glacial acetic acid; EDTA 1 mM, pH 8.0) displaying the DNA by staining with ethydium bromide (0.5 µg/ml).

The results obtained (Fig. 6B) confirm the correct integration ofthe B polynucleotide in the genome of the eukaryotic cell A549. The loss of the *attB* region confirms, furthermore, that said integration has been mediated specifically and unidirectionally by the PhiC31 integrase.

Then, and to determine the adenovirus gene expression, the pRc-1 clone was incubated in a 20 cm² plate at 37°C, 5% CO₂, until reaching a confluence of 80%. At this time, the cells were transfected with a 5 µg dilution of construction pGa14-2RU-SceI-E1, Plus *Reagent* (1:0.75) and 12 µl *Lipofectamine LTX,* following the aforementioned procedure. The transfections were carried out by duplicate incubating one of the plates in the presence of the RU486 inducer at a concentration of 10⁻⁸M. After 48 hours of incubation the cells were lysed with 1 ml of TRIZOL (Invitrogen) to proceed with the extraction of the total RNA total following the manufacturer's recommendations. This RNA was used for retrotranscription assays (RT)-PCR with specific primers for the adenoviral genes

| **Gene** | **Primer** | **Sequence** | **SEQ ID NO:** |
|---|---|---|---|
| **IVa2** | **PRC1** | 5'-TGATCCAGTCGTAGCAGGAG-3' | **35** |
| | **PRC4** | 5'-ACGATCTCATCCTGGAACAC-3' | **36** |
| **III** | **III1** | 5'-GCCAGTGGCGGCGGCGCTGGG-3' | **37** |
| | **III2** | 5'-CCGATGTCGCTTTCCAGAACC-3' | **38** |
| **E4** | **E4-1** | 5'-CGTGCGAGGTCTTCCCTGCAG-3' | **39** |
| **E4-2** | | 5'-CTACATGGGGGTAGAGTCATA-3' | **40** |

For these assays, 1 µg of RNA was previously treated with 1 unit of DNase (*DNasaI amplification grade;* Invitrogen) in reaction buffer (20 mM Tris-HCl, pH 8.4; 2 mM MgCl₂; 50 mM KCl) and at a final volume of 10 µl. The reaction was incubated at ambient temperature for 15 minutes and the DNase was inactivated by adding 1 µl of 25 mM EDTA solution, and incubating at 65 °C for 10 min. In the cDNA synthesis reaction, 625 ng of this DNA-free RNA were used. The reverse-transcription reaction was started mixing the sample with 10 pmol of the specific primer, 1 µl of dNTPs (10 mM) and water until a final volume of 13 µl. After incubating at 65 °C for 5 minutes, the mixture was cooled in ice and 2 µl of 0.1M DTT and 4 µl of reaction buffer (250 mM Tris-HCl, pH 8.3; 375 mM KCI; 15 mM MgCl₂) were used. Finally, after a new incubation at 37 °C for 2 minutes 200 U of *M-MLV Reverse-Transcriptase* (Invitrogen) and the incubation was continued at 37°C for 50 min. Next, the resulting cDNA was used as mould in the PCR reaction in order to amplify the transcription product of the genes under study. As negative control and to check the absence of contamination by genomic DNA, in all cases, an amplification reaction was included using as mould the RNA treated with DNase. The amplification conditions used for the PCR reaction with the cDNA obtained of the reverse-transcription reaction were the same as those used in the amplification reactions of the genomic DNA.

The results of these assays, shown in Figure 7, confirm the transcription capacity of the adenoviral genes stably integrated in the chromosome of the eukaryotic cell.

Finally, to confirm the capacity of the cells of producing viral proteins from the viral mRNA, the protein detection of the viral capsid was assayed by immunocytochemical assays. To induce protein synthesis, the pRc-1 clone was previously transfected with the construction pGa14-2RU-SceI-E1. The transfections were made in duplicate. The cells were incubated in the presence and absence of the RU486 inducer. After 24 hours of incubation, the cells were dettached with trypsin and 8x10⁴ cells were transferred to a four-cell cultureslide (*BD Falcon Cultureslide;* BD Biosciences, Erembodegem 9320, Belgium), adding new culture medium with and without inducer RU486. On the following day, the medium was removed and the cells were washed with PBS. Next, they were fixed with an acetone:methanol (1:1) solution previously cooled to -20 °C. The fixation solution was left to evaporate at ambient temperature (30 minutes) and the slides were immersed in PBS 1X. After 3 washings for 3 minutes in PBS, it was hybridized with the primary antibody (*Mouse anti-adenovirus monoclonal antibody blend;* Millipore Ibérica, 28050 Madrid, Spain) previously diluted in PBS (1:75). Said hybridization was carried out throughout the night at 4 °C, in darkness and wet chamber. The next day, the excess antibody was washed in PBS (3 x 3 minute washes) and it was hybridized with the secondary antibody (*Immunopure goat anti-rraouse IgG-Peroxidase;* Pierce Biotechnology, Rockford IL61105, USA) also diluted in PBS (1:400). The hybridization was incubated for 45 minutes at ambient temperature and in darkness. Again, the samples were washed 3 times in PBS 1X and they were developed with the DAB chromogen (*DakoCytomation Liquid DAB Substrate Chromogen System;* Dako Diagnósticos, Sant Just Desvern E-08960, Barcelona, Spain) following the manufacturer's recommendations. The samples were counter-stained with Mayer's Hematoxilin (5 seconds) for the specific staining of nuclei and, after a 15 minute wash in water, the samples were dehydrated by submerging them for 1 minute in 70%, 96% and 100% alcohol, and 10 minutes in xylene.

The brown colouring detected in the sample confirms the synthesis of proteins of the viral capsid. Although a slight basal production of these proteins is detected without inducer, the results of the immunocytochemistry reveal a clear difference with respect to the expression levels of viral proteins in the presence of the RU486 inducer, confirming that the mifepristone regulation system functions in the control of viral gene expression integrated in the genome of clone pRc-1.

### Example 3.2: Transfection of A549 cells with the pGa14-2RUSceI-E1 plasmid (polynucleotide A) for the generation of stable cell lines which integrate the E1 region and the I-SceI gene, both under control of the transcriptional regulation inducible by mifepristone.

In a second embodiment, a stable cell line was designed wherein the A2 polynucleotide has been integrated, as defined in the invention (Fig. 1A). Thus, the cell line thus obtained has the genes necessary for the start and control of the expression of the adenoviral genes included in the B polynucleotide. In turn, these genes, such as the gene which encodes the I-SceI restriction endonuclease and the E1 adenoviral region which encodes, in turn, E1A and E1B proteins, are under control of the promoters regulated by mifepristone. The regulation factor GAL4-hPR-p65 is included in the A2 polynucleotide and, therefore, it will be simultaneously integrated in the cell genome. Said transactivator will be constitutively expressed by the modified cells.

To obtain this cell line, A549 cells were incubated in a 10 cm² plates at 37 °C, 5% CO₂, until reaching a confluence of 80%. The cells were transfected by the lipofectamine method with the pGa14-2RUSceI-E1 construction (Zeomycin resistant gene (zeo^{r})) and following the procedure described in section 3.1 to obtain the pRc-1 clone. The cells were incubated in DMEM medium supplemented with 10% foetal bovine serum, 1% L-Glu and 1% P/S and the antibiotic Zeomycin at a concentration of 400 ug/ml. After successive amplification runs an antibiotic-resistant clone was isolated which received the name of Gal-13. As for the pRc-1 clone, the stability of the Gal-13 clone was tested for its maintenance in culture during a period of more than 4 months and more than 35 successive runs.

PCR assays from the genomic DNA of the cell confirmed the presence of the adenoviral genes E1a and E1b, of the gene encoding the I-SceI endonuclease and of the gene which encodes the fusion GAL4 protein- hPR-p65 regulating transcription and inducible by mifepristone. The primers used were:

| Gene | Primer | Sequence | SEQ ID NO: |
|---|---|---|---|
| E1a | RT-EA | 5'-TATCTGCCACGGAGGTGTTAT-3' | 41 |
| | RT-EA2 | 5'-TAGACAAACATGCCACAGGTC-3' | 42 |
| E1b | RT-EB3 | 5'-GCTTGGGAGTGTTTGGAAGAT-3' | 43 |
| | RT-EB4 | 5'-CTGCTCCTCCGTCGGTATTAT-3' | 44 |
| SceI | SceI-3 | 5'-CCATACGATGTTCCTGACTA-3' | 45 |
| | SceI-4 | 5'-CCCTTAACCAGGTATTCTACT-3' | 46 |
| GAL4- hPR- p65 | Gal-1 | 5'-CTGACTAGGGCACATCTGACA-3' | 47 |
| | Gal-2 | 5'-TTGATGAGCTCTCTAATGTAG-3' | 48 |

The genomic DNA of the cells was extracted with the *QIAmp DNA minikit* system (Qiagen) following the manufacturer's recommendations and the PCR reactions were carried out in presence of 1 U of the enzyme *Taq* polymerase (*BioTaq,* Bioline), PCR buffer (Bioline), 4 mM MgCl₂, 0.4 mM of each dNTP, 10 pmol of each primer, DNA and sterile deionized water until a final volume of 50 µl. The amplification were the following: 1 cycle of denaturing of 4 minutes at 95 °C; 29 cycles of 50 seconds at 94 °C of denaturing, 40 seconds at 50 °C of hybridization with the first and 40 seconds at 72 °C of elongation; 1 cycle of 5 minutes at 72 °C of final elongation. For the analysis of the amplification products the samples were mixed with 1.5 µl of the loading solution (0.25% bromophenol blue in distilled water; 40% Glycerol; 100 mM EDTA) and they were analysed by electrophoresis in agarose gels (1%) with TAE 1X buffer (40 mM Tris Base; 20 mM glacial acetic acid; 1 mM EDTA, pH 8.0) displaying the DNA by staining with ethydium bromide (0.5 µg/ml). The amplification results confirmed the correct integration of the genes included in the A2 polynucleotide within the genome of line A549 (Fig. 8).

Next, in order to check the regulation capacity of the gene expression of the system inducible by mifepristone, Western blot was used to analyze the synthesis of E1A and I-SceI proteins from proteins samples obtained from cultures incubated in the presence and absence of the RU486 inducer at concentration of 10⁻⁸M. The protein extracts were purified in 500 µl of *Passive Lysis Buffer* 1X (Promega) and the total protein levels were determined using Bradford's techniques for protein quantification (Bradford, 1976, *Anal. Biochem.,* 72: 248-254). The samples were analysed by electrophoresis in discontinuous polyacrylamide-SDS gel and *Laemli* electrophoresis buffer (25 mM Tris-HCl; 192 mM Glycine; 0.1 % w/v SDS). The resolving gel was composed of 12% polyacrylamide in 0.39 M Tris-HCl buffer (pH 8.8) with 0.1% SDS. As catalysts, 0.1% PSA and 0.94% TEMED were used. For its part, the stacking gel was composed of 5% polyacrylamide in 0.125 M Tris-HCl (pH6.8) with 0.1% SDS, 0.1% PSA and 0.01% TEMED. The samples were transferred to a *Hybond*-P membrane (GE Healthcare Europe) in transfer buffer (0.3% Tris-Base; 186 mM Glycine; 20% methanol) for 2 hours at 4 °C. Next, the membrane was blocked in a 5% milk solution in PBS+Tween (0.05%) at 4 °C throughout the night. The hybridization with the primary antibody was carried out in a 1% milk + PBS-Tween solution for 1.5 hours at ambient temperature. The excess antibody was eliminated with 3 x 10 minute washes in PBS-Tween. The hybridization with the secondary antibody was performed in 1% milk + PBS-Tween for 30 minutes at ambient temperature. In the detection of the E1A protein the antibody *Anti-adenovirus2 E1A (Mouse)* (1:500) (Calbiochem^{®}: EMD, La Jolla CA92039-2087, USA) was used and as secondary antibody *Immunopure goat anti-mouse IgG-Peroxidase* (1:5000) (Pierce Biotechnology). In the case of the I-SceI protein, as sole antibody *Anti-HA-Peroxidase High affinity* (1:500) (Roche Diagnostics, 08174 Barcelona, Spain) was used. After 3 washes of the membrane in PBS-Tween for 10 minutes, it was developed. To do this, the *Western lightning chemiluminescence reagent* system (Perkin-Elmer, 28760 Madrid, Spain) was used following the manufacturer's recommendations. The results obtained are shown in figure 9 and demonstrate the correct production of the proteins analysed as well as the increase in expression levels of these proteins in the presence of the inducer. However, it is necessary to take into account that, in the absence of inducer, there is a basal expression of the proteins subjected to regulation.

### Example 3.3: Generation of stable cell lines by double transfection of A549 cells with the Ga14-2RUSceI-E1 (polynucleotide A) and pRcAd2 (B polynucleotide) plasmids.

To obtain a unique cell line with all the necessary genes in the production of viral particles, the Zeomycin-resistant clone Gal-13 (example 3.2) was transfected with the pRcAd2 (neo^{r}) construction, which includes the rest of the adenovirus genes, and the pCMV-Int plasmid which encodes the PhiC31 integrase. The transfection conditions were the same as those used in obtaining the Gal-13 clone and the selection was made in the presence of the antibiotics G418 (600 µg/ml) and Zeomycin (400 µg/ml). This selection resulted in the isolation of the two double resistant clones (zeo^{r}+neo^{r}) and which were called GAd-1 and GAd-6 respectively. The morphological characteristics of both clones differ enormously from the parenteral line A549 and its derivative Gal-13. The new transfected clones tend to have spherical form and are of smaller size. This morphological change is possibly due to the basal transcription of the E1 genes previously detected in the Gal-13 clone, which entails the transcription of the rest of the adenoviral genes cloned in the pRcAd2 plasmid and inserted in the cell genome. However, the viral protein levels do not significantly affect the stability and viability of the cells, as has been verified after their maintenance in culture during a period greater than 4 months.

To confirm the integration of the pRcAd2 plasmid in the GAd-1 and GAd-6 clones, genomic DNA was extracted with the *QIAmp DNA minikit* system (Qiagen) following the manufacturer's recommendations. This DNA was used in nested-PCR assays to detect the presence of the adenoviral genes IIIa, IV, the gene encoding protease and the gene which encodes the viral polymerase. The primers used in this detection were the same as used in section 3.1. Likewise, the amplification reactions were carried out in the same conditions as those described in section 3.1.

The amplification products were analysed by electrophoresis in agarose gels (1%) with TAE IX buffer (40 mM Tris Base; 20 mM glacial acetic acid; 1 mM EDTA, pH 8.0) and visualizing the DNA by staining with ethydium bromide (0.5 µg/ml) (Fig. 10).

To confirm the synthesis of viral proteins, immunocytochemical assays were designed against the viral capsid. In this case, the GAd1 and GAd6 clones were incubated for 48 hours both in the presence and the absence of the RU486 inducer at a concentration of 10⁻⁸M. Next, 6x10⁴ cells were transferred to a four-well culture plate (*BD Falcon Cultureslide;* BD) and the same was performed as in the case of the pRc-1 cline (example 3.1). As primary antibody, the *Mouse anti-adenovirus monoclonal antibody blend* (Millipore) antibody was used diluted in PBS (1:75) and as secondary antibody the antibody *Immunopure goat anti-mouse IgG-Per-oxidase* (Pierce Biotechnology) also diluted in PBS (1:400).

The brown colouring detected in the sample confirms the synthesis of proteins of the viral capsid. However, no clear differences were detected between the samples incubated in the presence of the RU486 inducer and in the absence thereof. Therefore, the basal expression of the E1 proteins, which escape the control of the transcription regulator GAL4-hPR-p65, is sufficient to start the expression of the rest of the adenoviral genes integrated in the cell genome.

### Example 4: Production and packaging of infective particles of the KCZ gutless virus.

### Example 4.1: Production and packaging of the KCZ virus in the pRc-1 packaging line by co-transfection system with two plasmids.

In the present example, the clone selected from the cell line generated in example 3.1 was used for the production and packaging of high-capacity adenovirus or *gutless* adenovirus. This production system makes it possible to obtain viral extracts of high-capacity adenovirus without the need to use a *helper* virus as complement of the deleted genes in the pRcAd2 plasmid and stably integrated in the chromosome of the eukaryotic cell.'

In order to compensate the deficiency of the E1 region, the system requires the transfection of the cell line, in each one of the production steps, with the pGal4-2RU-SceI-E1 plasmid. This complementation plasmid expresses genes E1a and E1b from the inducible promoter regulated by mifepristone (Fig. 1A), which will start the expression of the rest of the adenoviral genes. Furthermore, the replication of the integrated viral genome will only be possible at the time of expression of the I-SceI endonuclease, included in the pGal4-2RU-SceI-E1 plasmid and also controlled on a transcriptional level by the GAL4-hPR-p65 regulator. The release of the viral genome and the start of its replication will permit obtaining multiple copies thereof and, therefore, sufficient levels of viral proteins for the encapsidation of the high-capacity adenovirus and its purification at a high titre.

To determine the system's capacity to produce infective particles, 8x10⁵ cells of the pRc-1 clone were incubated in a 20 cm² dish at 37 °C, 5% CO₂, until reaching a confluence of 80%. Next, the cells were co-transfected with 5 µg of the pGal4-2RU-SceI-E1 plasmid and 12 µg of the KCZ *gutless* vector, digested with the restriction enzyme *Pme*I to eliminate the replication origin, the ampicillin-resistant gene and leave the ITR ends of the vector free. As reporter gene, the KCZ vector carries the bacterial β-galactosidase gene (*lacZ*).

In the co-transfection, lipofectamine was used as system for the internalization of the DNA in the eukaryotic cell. Thus, the DNA of both plasmids was previously diluted in 1 ml of medium without *OPTI-MEM* (Gibco^{®}) serum. To enhance the formation of complexes between the DNA and cationic lipids, the *Plus Reagent* (Invitrogen) reagent was added in a 1:0.75 ratio. After 5 minutes of incubation at ambient temperature, 12 µl of *Lipofectamine LTX* (Invitrogen) were added and the mixture was again incubated at ambient temperature for 25 minutes. Next, the dilution was added drop by drop on the culture medium of the cells (DMEM supplemented with 10% foetal bovine serum). After 4 hours of incubation at 37 °C, 5% CO₂, the medium was replaced by fresh DMEM medium supplemented with 2% foetal bovine serum and RU486 inducer at a concentration of 10⁻⁸M. '

As no clear cytopathic effect was observed, the cells were incubated between 48 and 120 hours and, later, they were collected at different times in their own medium. After a centrifugation at 1250 rpm for 5 minutes, the precipitate was resuspended in 200 ul of 0.1M Tris-HCl (pH 8.0) and the cells were lysed by three cycles of freezing/thawing. The cell debris was centrifuged and the titre of the supernatant was determined by X-gal staining or 2935 cells infected with the preparations of the KCZ vector. To do this, the 293S cells were grown in 2 cm² wells until reaching a confluence of 80% in DMEM medium supplemented with 2% foetal bovine serum and 1% Na-Pyruvate. Next, the culture medium was removed and the cells were infected with serial dilutions of the viral extract in 2% DMEM + FBS + 1% Na-Pyruvate. After an incubation of 4 hours at 37 °C, 5% CO₂, fresh medium was added and the incubation was continued a further 48 hours. Finally the cells were fixated with a 0.5% glutaraldehyde solution in PBS for 10 minutes and at ambient temperature, they were washed 3 times in PBS and they were stained for 4 hours at 37 °C with 1 mg/ml of X-gal (5-bromo-4-chloro-3-indolyl-(3-galactopyranoside) in PBS supplemented with 20 mM Much, 5 mM K₃Fe(CN₆) and 5 mM K₄Fe(CN₆).

According to the results obtained, when using the pRc-1 clone as production and packaging line, the production to passage 0 reaches its maximum levels after 48-72 hours and are around 7x10⁵ infective units (ui)/ml (Fig. 11). In the successive amplification runs, these extracts were used for the infection of new pRc-1 cells, previously transfected with the pGal4-2RU-Scel-El plasmid. The described system permits, therefore, the recovery of extracts of high-capacity adenovirus free from infective particles derived from a *helper* virus, as well as their obtainment at high production levels.

### Example 4.2: Production and packaging of the KCZ virus in cells of the GAd-1 and GAd-6 clones. Unique transfection system with the high-capacity vector.

The production system described below uses as packaging cell a modified line wherein the A2 and B polynucleotides described in the invention have been stably integrated (Fig. 1A, 1B). Except for the polynucleotide corresponding to the high-capacity adenoviral vector, this cell line does not require any additional element (plasmid or virus *helper*) for the production of viral particles with infective capacity.

The system integrates the adenoviral genes necessary for the encapsidation and replication of the high-capacity adenovirus (B polynucleotide), as well as the genes of the E1 region and the I-SceI restriction endonuclease (A2 polynucleotide), necessary for the controlled expression at high levels of the previous viral proteins, in a single cell. Finally, the start of the production process is regulated by the expression of the genes of the E1 region and the restriction endonuclease from its corresponding inducible promoters and the transcriptional activator GAL4-hPR-p65.

In the present example of infective particle production, the GAd-6 and GAd-1 packaging lines were transfected with 12 µg of the KCZ *gutless* vector which encodes the reporter gene of β-galactosidase and which has been previously linearized with the *Pme*I restriction enzyme, as indicated in example 4.1. The transfection with the high-capacity vector was carried out following the procedure described in the production system with the pRc-1 packaging line (example 4.1).

The cells were incubated between 48 and 120 hours in DMEM medium supplemented with 2% foetal bovine serum and in presence of the RU486 inducer at a concentration of 10⁻⁸M. The cells were collected after different times, they were centrifuged at 1250 rpm for 5 minutes and, finally, they were resuspended in 200 µl of 0.1 M Tris-HCl. After its lysis with three cycles of freezing/thawing, the cell residues were centrifuged and the supernatant was titrated in the 293S cell line following the protocol described in the previous example.

Using the GAd-1 clone as production and packaging line, the production levels at passage 0 were of up to 1.7x10⁶ infective units (ui/ml), whilst for the GAd-6 clone a titration of 3x10⁶ ui/ml (Fig. 12) was obtained.

As in the previous example, these extracts were used for the infection of new packaging cells during the successive amplification runs. The system described in the present embodiment differs from the previous system of co-transfection with two plasmids (example 4.1) or other *helper*-dependent systems by the elimination of the transfection/infection process of the packaging cells in each one of the virus amplification runs. This characteristic sufficiently reduces the production time and increases the yield thereof. The other great advantage is the absence of contaminations with *helper* virus, which simplifies the virus purification process and permits the production of sufficient quantities for its use in gene therapy procedures in humans.

## Claims

1. A polynucleotide or expression vector which comprises a nucleotidic sequence which comprises the genome of a recombinant adenovirus which lacks the ψ packaging sequence and the sequence which encodes the E1A protein or E1A/E1B protein, wherein said sequence which comprises the genome of a recombinant adenovirus is flanked by recognition sequences specific for a restriction endonuclease.

2. Polynucleotide or expression vector according to claim 1, further comprising a nucleotidic sequence for specific recognition by an integrase, where the integrase recognition sequence is in position 5' with respect to the first recognition sequence for the restriction endonuclease.

3. Polynucleotide or expression vector according to claim 2, wherein the nucleotidic sequence for recognition by the integrase is *attB,* specific for PhiC31 integrase.

4. Polynucleotide or expression vector according to any of claims 1 to 3, wherein the recognition sequences for the endonuclease are specific for the I-SceI endonuclease.

5. A host cell which comprises a polynucleotide or expression vector according to any of claims 1 to 4.

6. A host cell selected from the group consisting of
(i) a host cell which comprises a polynucleotide or expression vector comprising
(a) a first expression cassette which comprises a nucleotidic sequence which encodes a transcriptional regulator;
(b) a second expression cassette which comprises a nucleotidic sequence which encodes a restriction endonuclease, and which is under the operational control of a transcriptional regulatory sequence activatable by the transcriptional regulator encoded by the polynucleotide defined in (a);
(ii) a host cell according to (i) which comprises, additionally, a sequence which encodes the adenoviral E1A protein and, optionally, a sequence which encodes the adenoviral E1B protein and
(iii) a host cell which comprises a polynucleotide or expression vector comprising
(a) a first expression cassette which comprises a nucleotidic sequence which encodes a transcriptional regulator;
(b) a second expression cassette which comprises a nucleotidic sequence which encodes a restriction endonuclease which is under the operational control of a transcriptional regulatory sequence activatable by the transcriptional regulator (a) and
(c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a),
wherein said host cell comprises, additionally, a polynucleotide or expression vector according to any of claims 1 to 4, or which can be obtained by integration in said host cell defined in (i), (ii) or (iii), of a polynucleotide according to claims 1 to 4, wherein the restriction targets which flank the viral genome derived from the polynucleotide or expression vector according to any of claims 1 to 4 are specifically recognized by the endonuclease restriction encoded by said second expression cassette.

7. A method for the production of cells suitable for the production and packaging of infective particles of a high-capacity adenovirus which comprises:
(i) transfecting a cell with
- a first polynucleotide comprising
(a) a first expression cassette which comprises a nucleotidic sequence which encodes a transcriptional regulator;
(b) a second expression cassette which comprises a nucleotidic sequence which encodes a restriction endonuclease, and which is under the operational control of a transcriptional regulatory sequence activatable by the transcriptional regulator encoded by the polynucleotide defined in (a); or
- a vector which comprises said first polynucleotide, or
- a second polynucleotide comprising
(a) a first expression cassette which comprises a nucleotidic sequence which encodes a transcriptional regulator;
(b) a second expression cassette which comprises a nucleotidic sequence which encodes a restriction endonuclease which is under the operational control of a transcriptional regulatory sequence activatable by the transcriptional regulator (a) and
(c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a), or
- a vector which comprises said second polynucleotide and
(ii) transfecting said cell with a polynucleotide according to any of claims 1 to 4 or with a vector which comprises said polynucleotide
wherein stages (i) and (ii) can be carried out in any order or simultaneously and wherein if the cells are transfected in stage (i) with the first polynucleotide or with a vector comprising said first polynucleotide, then cells are used which comprise the sequence which encodes the adenoviral E1A protein and, optionally, the adenoviral E1B protein.

8. Method according to claim 7, wherein stages (i) and/or (ii) include an additional selection step wherein cells are selected which have stably integrated the first and/or the second polynucleotide based on selection markers present in the polynucleotides used in stages (i) and (ii).

9. Method according to claims 7 or 8, wherein the polynucleotide according to any of claims 1 to 4 additionally comprises a nucleotidic sequence for specific recognition by an integrase and wherein the transfection performed in stage (ii) includes, additionally, a polynucleotide which encodes an integrase specific for said recognition sequences.

10. Method according to claim 9, wherein the specific integrase is the integrase of *Streptomyces* PhiC31 phage and wherein the specific recognition site is an *attB* site.

11. Host cell according to claim 6 or method according to any of claims 7 to 10, where the transcriptional regulator encoded by cassette (a) is an activatable transcriptional regulator.

12. Host cell or method according to claim 11, where the activatable transcriptional regulator (a) is formed by the DNA binding domain of GAL4, the ligand binding domain of the human progesterone receptor and by the activating domain of NF-kappaB p65.

13. Host cell according to claims 6, 11 or 12, or method according to claims 7 to 12, where the restriction endonuclease encoded by cassette (b) is I-SceI.

14. Cell which can be produced by a method according to any of claims 7 to 13.

15. A system for the production of high-capacity adenovirus which comprises
(a) a cell according to any of claims 6, 11, 13 or 14 and
(b) a high-capacity adenovirus.

16. Method for the production of high-capacity adenoviral vectors, which comprises the stages of:
(a) transfecting or infecting a host cell with a polynucleotide which comprises the genome of the high-capacity adenovirus which production is intended or with an expression vector which comprises said polynucleotide,
(b) cultivating the transfected/infected cells under conditions adequate for allowing the replication and packaging of the high-capacity adenovirus,
(c) recovering the viral particles of the high-capacity adenovirus and, optionally,
(d) repeating stages (a) to (c) wherein the high-capacity viral particles produced in stage (c) are used in stage (a) of the following cycle, wherein the host cell used in stage (a) is selected from the group of
(i) a cell which comprises
- a polynucleotide comprising
(a) a first expression cassette which comprises a nucleotidic sequence which encodes a transcriptional regulator;
(b) a second expression cassette which comprises a nucleotidic sequence which encodes a restriction endonuclease which is under the operational control of a transcriptional regulatory sequence activatable by the transcriptional regulator (a)
- the nucleotidic sequence which encodes the adenoviral E1A protein and, optionally,
- the adenoviral E1B protein,
in which case stage (a) is preceded by or carried out simultaneously with a stage of transfection with a polynucleotide according to claims 1 to 4 and/or an expression vector which comprises said polynucleotide,
(ii) a cell which comprises a polynucleotide comprising
(a) a first expression cassette which comprises a nucleotidic sequence which encodes a transcriptional regulator;
(b) a second expression cassette which comprises a nucleotidic sequence which encodes a restriction endonuclease which is under the operational control of a transcriptional regulatory sequence activatable by the transcriptional regulator (a) and
(c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a),
in which case stage (a) is preceded by or carried out simultaneously with a stage of transfection with a polynucleotide according to claims 1 to 4 and/or an expression vector which comprises said polynucleotide,
(iii) a cell which comprises a polynucleotide according to any of claims 1 to 4, in which case stage (a) is preceded by or carried out simultaneously with a stage of transfection with a polynucleotide comprising
(a) a first expression cassette which comprises a nucleotidic sequence which encodes a transcriptional regulator;
(b) a second expression cassette which comprises a nucleotidic sequence which encodes a restriction endonuclease, and which is under the operational control of a transcriptional regulatory sequence activatable by the transcriptional regulator encoded by the polynucleotide defined in (a),
and wherein the cell comprises a sequence which encodes the adenoviral E1A protein and, optionally, the adenoviral E 1 B protein,
(iv) a cell which comprises a polynucleotide according to any of the claims 1 to 4, in which case stage (a) is preceded by or carried out simultaneously with a stage of transfection with
- a polynucleotide comprising
(a) a first expression cassette which comprises a nucleotidic sequence which encodes a transcriptional regulator;
(b) a second expression cassette which comprises a nucleotidic sequence which encodes a restriction endonuclease which is under the operational control of a transcriptional regulatory sequence activatable by the transcriptional regulator (a) and
(c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a), and/or
- an expression vector which comprises said polynucleotide,
(v) a cell which comprises
- a polynucleotide comprising
(a) a first expression cassette which comprises a nucleotidic sequence which encodes a transcriptional regulator;
(b) a second expression cassette which comprises a nucleotidic sequence which encodes a restriction endonuclease, and which is under the operational control of a transcriptional regulatory sequence activatable by the transcriptional regulator encoded by the polynucleotide defined in (a),
- a polynucleotide according to claims 1 to 4 and
- a sequence which encodes the adenoviral E1A protein and, optionally, the adenoviral E1B protein, and
(vi) a cell which comprises
- a polynucleotide comprising
(a) a first expression cassette which comprises a nucleotidic sequence which encodes a transcriptional regulator;
(b) a second expression cassette which comprises a nucleotidic sequence which encodes a restriction endonuclease which is under the operational control of a transcriptional regulatory sequence activatable by the transcriptional regulator (a) and
(c) a third expression cassette which comprises a nucleotidic sequence which encodes the adenoviral E1A protein or the adenoviral E1A and E1B proteins, and which is under the operational control of a transcription regulatory sequence activatable by the transcriptional regulator (a), and
- a polynucleotide according to any of claims 1 to 4.

17. Method according to claim 16, where the culture of stage (b) is performed in the presence of a specific inducer agent of the transcriptional regulator encoded by said first expression cassetten

18. Method according to claim 17, wherein the transcription regulator is formed by the DNA binding domain of GAL4, the ligand binding domain of human progesterone receptor and by the activating domain of NF-kappaB p65 and where the inducer agent is a synthetic antiprogestin, preferably mifepristone.

19. Method according to any of claims 16 to 18 where the transcriptional regulator encoded by cassette (a) is an activatable transcriptional regulator.

20. Method according to claim 19, where the activatable transcriptional regulator (a) is formed by the DNA binding domain of GAL4, the ligand binding domain of the human progesterone receptor and by the activating domain of NF-kappaB p65.

21. Method according to any of claims 16 to 20, where the restriction endonuclease encoded by cassette (b) is I-SceI.

## Patentansprüche

1. Polynucleotid oder Expressionsvektor, der eine Nucleotidsequenz umfasst, die das Genom eines rekombinanten Adenovirus umfasst, dem die Ψ-Packsequenz und die Sequenz fehlt, die das E1A-Protein oder E1A/E1B-Protein codiert, wobei die Sequenz, die das Genom eines rekombinanten Adenovirus umfasst, durch Erkennungssequenzen flankiert wird, die für eine Restriktionsendonuclease spezifisch sind.

2. Polynucleotid oder Expressionsvektor nach Anspruch 1, die/der des Weiteren eine Nucleotidsequenz für die spezifische Erkennung durch eine Integrase umfasst, wobei sich die Integrase-Erkennungssequenz an Position 5' in Bezug auf die erste Erkennungssequenz für die Restriktionsendonuclease befindet.

3. Polynucleotid oder Expressionsvektor nach Anspruch 2, wobei die Nucleotidsequenz für die Erkennung durch die Integrase *attB* ist, die für die PhiC31-Integrase spezifisch ist.

4. Polynucleotid oder Expressionsvektor nach einem der Ansprüche 1 bis 3, wobei die Erkennungssequenzen spezifisch für die Endonuclease für die 1-Scel-Endonuclease sind.

5. Wirtszelle, die ein Polynucleotid oder einen Expressionsvektor nach einem der Ansprüche 1 bis 4 umfasst.

6. Wirtszelle, ausgewählt aus der Gruppe bestehend aus
(i) einer Wirtszelle, die ein Polynucleotid oder einen Expressionsvektor umfasst, umfassend
(a) eine erste Expressionskassette, die eine Nucleotidsequenz umfasst, die einen transkriptionellen Regulator codiert;
(b) eine zweite Expressionskassette, die eine Nucleotidsequenz umfasst, die eine Restriktionsendonuclease codiert, und die unter der operativen Kontrolle einer transkriptionellen Regulatorsequenz ist, die durch den transkriptionellen Regulator aktivierbar ist, der durch das in (a) definierte Polynucleotid codiert wird;
(ii) einer Wirtszelle nach (i), die zusätzlich eine Sequenz umfasst, die das adenovirale E1A-Protein codiert und, gegebenenfalls, eine Sequenz, die das adenovirale E1B-Protein codiert, und
(iii) einer Wirtszelle, die ein Polynucleotid oder einen Expressionsvektor umfasst, umfassend
(a) eine erste Expressionskassette, die eine Nucleotidsequenz umfasst, die einen transkriptionellen Regulator codiert;
(b) eine zweite Expressionskassette, die eine Nucleotidsequenz umfasst, die eine Restriktionsendonuclease codiert, die unter der operativen Kontrolle einer transkriptionellen Regulatorsequenz ist, die durch den transkriptionellen Regulator (a) aktivierbar ist, und
(c) eine dritte Expressionskassette, die eine Nucleotidsequenz umfasst, die das adenovirale E1A-Protein oder die adenoviralen E1A- und E1 B-Proteine codiert, und die unter der operativen Kontrolle einer transkriptionellen Regulatorsequenz ist, die durch den transkriptionellen Regulator (a) aktivierbar ist,
wobei die Wirtszelle zusätzlich ein Polynucleotid oder einen Expressionsvektor nach einem der Ansprüche 1 bis 4 umfasst, oder die erhalten werden kann durch Integration in die in (i), (ii) oder (iii) definierte Wirtszelle eines Polynucleotids nach einem der Ansprüche 1 bis 4, wobei die Restriktionsziele, die das virale Genom, das von dem Polynucleotid oder dem Expressionsvektor nach einem der Ansprüche 1 bis 4 stammt, flankieren, von der Restriktionsendonuclease, die von der zweiten Expressionskassette codiert wird, spezifisch erkannt werden.

7. Verfahren zur Herstellung von Zellen, die für die Herstellung und das Verpacken von infektiösen Partikeln eines Adenovirus hoher Kapazität geeignet sind, das umfasst:
(i) Transfizieren einer Zelle mit
- einem ersten Polynucleotid, umfassend
(a) eine erste Expressionskassette, die eine Nucleotidsequenz umfasst, die einen transkriptionellen Regulator codiert;
(b) eine zweite Expressionskassette, die eine Nucleotidsequenz umfasst, die eine Restriktionsendonuclease codiert, und die unter der operativen Kontrolle einer transkriptionellen Regulatorsequenz ist, die durch den transkriptionellen Regulator aktivierbar ist, der durch das in (a) definierte Polynucleotid codiert wird; oder
- einem Vektor, der das erste Polynucleotid umfasst, oder
- einem zweiten Polynucleotid, umfassend
(a) eine erste Expressionskassette, die eine Nucleotidsequenz umfasst, die einen transkriptionellen Regulator codiert;
(b) eine zweite Expressionskassette, die eine Nucleotidsequenz umfasst, die eine Restriktionsendonuclease codiert, die unter der operativen Kontrolle einer transkriptionellen Regulatorsequenz ist, die durch den transkriptionellen Regulator (a) aktivierbar ist, und
(c) eine dritte Expressionskassette, die eine Nucleotidsequenz umfasst, die das adenovirale E1A-Protein oder die adenoviralen E1A- und E1B-Proteine codiert, und die unter der operativen Kontrolle einer transkriptionellen Regulatorsequenz ist, die durch den transkriptionellen Regulator (a) aktivierbar ist, oder
- einem Vektor, der das zweite Polynucleotid umfasst, und
(ii) Transfizieren der Zelle mit einem Polynucleotid nach einem der Ansprüche 1 bis 4 oder mit einem Vektor, der das Polynucleotid umfasst,
wobei die Stadien (i) und (ii) in beliebiger Reihenfolge oder simultan durchgeführt werden können, und wobei, falls die Zellen in Stadium (i) mit dem ersten Polynucleotid oder mit dem Vektor, der das erste Polynucleotid umfasst, transfiziert werden, dann Zellen verwendet werden, die die Sequenz umfassen, die das adenovirale E1A-Protein und, gegebenenfalls, das adenovirale E1 B-Protein codiert.

8. Verfahren nach Anspruch 7, wobei die Stadien (i) und/oder (ii) einen zusätzlichen Selektionsschritt einschließen, wobei Zellen selektiert werden, die das erste und/oder das zweite Polynucleotid stabil integriert haben, basierend auf den Selektionsmarkern, die in den Polynucleotiden vorhanden sind, die in den Stadien (i) und (ii) verwendet wurden.

9. Verfahren nach Anspruch 7 oder 8, wobei das Polynucleotid nach einem der Ansprüche 1 bis 4 zusätzlich eine Nucleotidsequenz für die spezifische Erkennung durch eine Integrase umfasst, und wobei die in Stadium (ii) durchgeführte Transfektion zusätzlich ein Polynucleotid einschließt, das eine Integrase codiert, die für die Erkennungssequenzen spezifisch ist.

10. Verfahren nach Anspruch 9, wobei die spezifische Integrase die Integrase des *Streptomyces* PhiC31-Phagen ist, und wobei die spezifische Erkennungsstelle eine *attB*-Stelle ist.

11. Wirtszelle nach Anspruch 6 oder Verfahren nach einem der Ansprüche 7 bis 10, wobei der durch Kassette (a) codierte transkriptionelle Regulator ein aktivierbarer transkriptioneller Regulator ist.

12. Wirtszelle oder Verfahren nach Anspruch 11, wobei der aktivierbare transkriptionelle Regulator (a) von der DNA-bindenden Domäne von GAL4, der Liganden-bindenden Domäne des menschlichen Progesteronrezeptors und von der aktivierenden Domäne von NF-kappaB p65 gebildet wird.

13. Wirtszelle nach Anspruch 6, 11 1 oder 12, oder Verfahren nach den Ansprüchen 7 bis 12, wobei die von Kassette (b) codierte Restriktionsendonuclease I-Scel ist.

14. Zelle, die durch ein Verfahren nach einem der Ansprüche 7 bis 13 hergestellt werden kann.

15. System zur Herstellung eines Adenovirus hoher Kapazität, das umfasst
(a) eine Zelle nach einem der Ansprüche 6, 11, 13 oder 14, und
(b) ein Adenovirus hoher Kapazität.

16. Verfahren zur Herstellung adenoviraler Vektoren hoher Kapazität, das die Stadien umfasst:
(a) Transfizieren oder Infizieren einer Wirtszelle mit einem Polynucleotid, das das Genom des Adenovirus hoher Kapazität umfasst, dessen Herstellung beabsichtigt ist, oder mit einem Expressionsvektor, der das Polynucleotid umfasst,
(b) Züchten der transfizierten/infizierten Zellen unter Bedingungen, die angemessen sind, um die Replikation und das Verpacken des Adenovirus hoher Kapazität zu erlauben,
(c) Gewinnen der viralen Partikel des Adenovirus hoher Kapazität und, gegebenenfalls,
(d) Wiederholen der Stadien (a) bis (c), wobei die in Stadium (c) hergestellten viralen Partikel hoher Kapazität in Stadium (a) des folgenden Zyklus verwendet werden,
wobei die in Stadium (a) verwendete Wirtszelle ausgewählt ist aus der Gruppe bestehend aus
(i) einer Zelle, umfassend
- ein Polynucleotid, umfassend
(a) eine erste Expressionskassette, die eine Nucleotidsequenz umfasst, die einen transkriptionellen Regulator codiert;
(b) eine zweite Expressionskassette, die eine Nucleotidsequenz umfasst, die eine Restriktionsendonuclease codiert, die unter der operativen Kontrolle einer transkriptionellen Regulatorsequenz ist, die durch den transkriptionellen Regulator (a) aktivierbar ist,
- die Nucleotidsequenz, die das adenovirale E1A-Protein codiert, und, gegebenenfalls,
- das adenovirale E1B-Protein,
in welchem Fall Stadium (a) nach oder gleichzeitig mit einem Stadium der Transfektion mit einem Polynucleotid nach Anspruch 1 bis 4 und/oder mit einem Expressionsvektor, der das Polynucleotid umfasst, ausgeführt wird,
(ii) einer Zelle, die ein Polynucleotid umfasst, umfassend
(a) eine erste Expressionskassette, die eine Nucleotidsequenz umfasst, die einen transkriptionellen Regulator codiert;
(b) eine zweite Expressionskassette, die eine Nucleotidsequenz umfasst, die eine Restriktionsendonuclease codiert, die unter der operativen Kontrolle einer transkriptionellen Regulatorsequenz ist, die durch den transkriptionellen Regulator (a) aktivierbar ist, und
(c) eine dritte Expressionskassette, die eine Nucleotidsequenz umfasst, die das adenovirale E1A-Protein oder die adenoviralen E1A- und E1B-Proteine umfasst, und die unter der operativen Kontrolle einer transkriptionellen Regulatorsequenz ist, die durch den transkriptionellen Regulator (a) aktivierbar ist,
in welchem Fall Stadium (a) nach oder gleichzeitig mit einem Stadium der Transfektion mit einem Polynucleotid nach Anspruch 1 bis 4 und/oder mit einem Expressionsvektor, der das Polynucleotid umfasst, ausgeführt wird,
(iii) einer Zelle, die ein Polynucleotid nach einem der Ansprüche 1 bis 4 umfasst, in welchem Fall Stadium (a) nach oder gleichzeitig mit einem Stadium der Transfektion mit einem Polynucleotid ausgeführt wird, umfassend
(a) eine erste Expressionskassette, die eine Nucleotidsequenz umfasst, die einen transkriptionellen Regulator codiert;
(b) eine zweite Expressionskassette, die eine Nucleotidsequenz umfasst, die eine Restriktionsendonuclease codiert, und die unter der operativen Kontrolle einer transkriptionellen Regulatorsequenz ist, die durch den transkriptionellen Regulator (a) aktivierbar ist, der durch das in (a) definierte Polynucleotid codiert wird,
und wobei die Zelle eine Sequenz umfasst, die das adenovirale E1A-Protein und, gegebenenfalls, das adenovirale E1 B-Protein codiert,
(iv) einer Zelle, die ein Polynucleotid nach einem der Ansprüche 1 bis 4 umfasst, in welchem Fall Stadium (a) nach oder gleichzeitig mit einem Stadium der Transfektion mit
- einem Polynucleotid, umfassend
(a) eine erste Expressionskassette, die eine Nucleotidsequenz umfasst, die einen transkriptionellen Regulator codiert;
(b) eine zweite Expressionskassette, die eine Nucleotidsequenz umfasst, die eine Restriktionsendonuclease codiert, und die unter der operativen Kontrolle einer transkriptionellen Regulatorsequenz ist, die durch den transkriptionellen Regulator (a) aktivierbar ist, und
(c) eine dritte Expressionskassette, die eine Nucleotidsequenz umfasst, die das adenovirale E1A-Protein oder die adenoviralen E1A- und E1B-Proteine umfasst, und die unter der operativen Kontrolle einer transkriptionellen Regulatorsequenz ist, die durch den transkriptionellen Regulator (a) aktivierbar ist, und/oder
- einem Expressionsvektor, der das Polynucleotid umfasst, ausgeführt wird,
(v) einer Zelle, umfassend
- ein Polynucleotid, umfassend
(a) eine erste Expressionskassette, die eine Nucleotidsequenz umfasst, die einen transkriptionellen Regulator codiert;
(b) eine zweite Expressionskassette, die eine Nucleotidsequenz umfasst, die eine Restriktionsendonuclease codiert, und die unter der operativen Kontrolle einer transkriptionellen Regulatorsequenz ist, die durch den transkriptionellen Regulator aktivierbar ist, der durch das in (a) definierte Polynucleotid codiert wird,
- ein Polynucleotid nach den Ansprüchen 1 bis 4 und
- eine Sequenz, die das adenovirale E1A-Protein und, gegebenenfalls, das adenovirale E1B-Protein codiert, und
(vi) einer Zelle, umfassend
- ein Polynucleotid, umfassend
(a) eine erste Expressionskassette, die eine Nucleotidsequenz umfasst, die einen transkriptionellen Regulator codiert;
(b) eine zweite Expressionskassette, die eine Nucleotidsequenz umfasst, die eine Restriktionsendonuclease codiert, die unter der operativen Kontrolle einer transkriptionellen Regulatorsequenz ist, die durch den transkriptionellen Regulator (a) aktivierbar ist, und
(c) eine dritte Expressionskassette, die eine Nucleotidsequenz umfasst, die das adenovirale E1A-Protein oder die adenoviralen E1A- und E1B-Proteine umfasst, und die unter der operativen Kontrolle einer transkriptionellen Regulatorsequenz ist, die durch den transkriptionellen Regulator (a) aktivierbar ist, und
- ein Polynucleotid nach einem der Ansprüche 1 bis 4.

17. Verfahren nach Anspruch 16, wobei das Züchten in Stadium (b) in der Anwesenheit eines spezifischen Inducer-Wirkstoffs des transkriptionellen Regulators durchgeführt wird, der von der ersten Expressionskassette codiert wird.

18. Verfahren nach Anspruch 17, wobei der transkriptionelle Regulator durch die DNA-bindende Domäne von GAL4, die Liganden-bindende Domäne des menschlichen Progesteron-Rezeptors und durch die aktivierende Domäne von NF-kappaB p65 gebildet wird, und wobei der Inducer-Wirkstoff ein synthetisches Antiprogestin, vorzugsweise Mifepriston ist.

19. Verfahren nach einem der Ansprüche 16 bis 18, wobei der transkriptionelle Regulator, der von Kassette (a) codiert wird, ein aktivierbarer transkriptioneller Regulator ist.

20. Verfahren nach Anspruch 19, wobei der aktivierbare transkriptionelle Regulator (a) durch die DNA-bindende Domäne von GAL4, die Liganden-bindenden Domäne des menschlichen Progesteron-Rezeptors und durch die aktivierende Domäne von NK-kappaB p65 gebildet wird.

21. Verfahren nach einem der Ansprüche 16 bis 20, wobei die Restriktionsendonuclease, die von Kassette (b) codiert wird, I-Scel ist.

## Revendications

1. Polynucléotide ou vecteur d'expression qui comprend une séquence nucléotidique qui comprend le génome d'un adénovirus recombinant dépourvu de la séquence d'encapsidation Ψ et de la séquence qui code la protéine E1A ou la protéine E1A/E1B, dans lequel ladite séquence qui comprend le génome d'un adénovirus recombinant est bordée par des séquences de reconnaissance spécifiques pour une endonucléase de restriction.

2. Polynucléotide ou vecteur d'expression selon la revendication 1, comprenant en outre une séquence nucléotidique pour la reconnaissance spécifique par une intégrase, où la séquence de reconnaissance de l'intégrase se trouve en position 5' par rapport à la première séquence de reconnaissance pour l'endonucléase de restriction.

3. Polynucléotide ou vecteur d'expression selon la revendication 2, dans lequel la séquence nucléotidique pour la reconnaissance par l'intégrase est *attB,* spécifique de l'intégrase PhiC31.

4. Polynucléotide ou vecteur d'expression selon l'une quelconque des revendications 1 à 3, dans lequel les séquences de reconnaissance pour l'endonucléase sont spécifiques pour l'endonucléase I-SceI.

5. Cellule hôte qui comprend un polynucléotide ou un vecteur d'expression selon l'une quelconque des revendications 1 à 4.

6. Cellule hôte sélectionnée dans le groupe constitué par
(i) une cellule hôte qui comprend un polynucléotide ou un vecteur d'expression comprenant
(a) une première cassette d'expression qui comprend une séquence nucléotidique qui code pour un régulateur transcriptionnel ;
(b) une deuxième cassette d'expression qui comprend une séquence nucléotidique qui code pour une endonucléase de restriction, et qui est sous le contrôle opérationnel d'une séquence régulatrice transcriptionnelle activable par le régulateur transcriptionnel codé par le polynucléotide défini en (a) ;
(ii) une cellule hôte selon (i) qui comprend, en outre, une séquence qui code la protéine E1A adénovirale et, facultativement, une séquence qui code la protéine E1B adénovirale et
(iii) une cellule hôte qui comprend un polynucléotide ou un vecteur d'expression comprenant
(a) une première cassette d'expression qui comprend une séquence nucléotidique qui code pour un régulateur transcriptionnel ;
(b) une deuxième cassette d'expression qui comprend une séquence nucléotidique qui code une endonucléase de restriction qui est sous le contrôle opérationnel d'une séquence régulatrice transcriptionnelle activable par le régulateur transcriptionnel (a) et
(c) une troisième cassette d'expression qui comprend une séquence nucléotidique qui code la protéine E1A adénovirale ou les protéines E1A et E1B adénovirales, et qui est sous le contrôle opérationnel d'une séquence régulatrice transcriptionnelle activable par le régulateur transcriptionnel (a),
dans laquelle ladite cellule hôte comprend, en outre, un polynucléotide ou un vecteur d'expression selon l'une quelconque des revendications 1 à 4, ou qui peut être obtenue par intégration dans ladite cellule hôte définie en (i), (ii) ou (iii), d'un polynucléotide selon les revendications 1 à 4, dans laquelle les cibles de restriction qui bordent le génome viral dérivé du polynucléotide ou du vecteur d'expression selon l'une quelconque des revendications 1 à 4 sont reconnues spécifiquement par l'endonucléase de restriction codée par ladite deuxième cassette d'expression.

7. Procédé de production de cellules approprié pour la production et l'encapsidation de particules infectieuses d'un adénovirus de grande capacité qui comprend :
(i) la transfection d'une cellule avec
- un premier polynucléotide comprenant
(a) une première cassette d'expression qui comprend une séquence nucléotidique qui code un régulateur transcriptionnel ;
(b) une deuxième cassette d'expression qui comprend une séquence nucléotidique qui code une endonucléase de restriction, et qui est sous le contrôle opérationnel d'une séquence régulatrice transcriptionnelle activable par le régulateur transcriptionnel codé par le polynucléotide défini en (a) ; ou
- un vecteur qui comprend ledit premier polynucléotide, ou
- un deuxième polynucléotide comprenant
(a) une première cassette d'expression qui comprend une séquence nucléotidique qui code un régulateur transcriptionnel ;
(b) une deuxième cassette d'expression qui comprend une séquence nucléotidique qui code une endonucléase de restriction qui est sous le contrôle opérationnel d'une séquence régulatrice transcriptionnelle activable par le régulateur transcriptionnel (a) et
(c) une troisième cassette d'expression qui comprend une séquence nucléotidique qui code la protéine E1A adénovirale ou les protéines E1A et E1B adénovirales, et qui est sous le contrôle opérationnel d'une séquence régulatrice transcriptionnelle activable par le régulateur transcriptionnel (a), ou
- un vecteur qui comprend ledit deuxième polynucléotide et
(ii) la transfection de ladite cellule avec un polynucléotide selon l'une quelconque des revendications 1 à 4 ou avec un vecteur qui comprend ledit polynucléotide,
dans lequel les étapes (i) et (ii) peuvent être réalisées dans n'importe quel ordre ou simultanément et dans lequel si les cellules sont transfectées à l'étape (i) avec le premier polynucléotide ou avec un vecteur comprenant ledit premier polynucléotide, sont alors utilisées les cellules qui comprennent la séquence qui code la protéine E1A adénovirale et, facultativement, la protéine E1B adénovirale.

8. Procédé selon la revendication 7, dans lequel les étapes (i) et/ou (ii) comprennent une étape de sélection supplémentaire dans laquelle sont sélectionnées les cellules qui ont intégré de manière stable le premier et/ou le deuxième polynucléotide sur la base de marqueurs de sélection présents dans les polynucléotides utilisés dans les étapes (i) et (ii).

9. Procédé selon les revendications 7 ou 8, dans lequel le polynucléotide selon l'une quelconque des revendications 1 à 4 comprend en outre une séquence nucléotidique pour la reconnaissance spécifique par une intégrase et dans lequel la transfection effectuée à l'étape (ii) comprend, en outre, un polynucléotide qui code une intégrase spécifique pour lesdites séquences de reconnaissance.

10. Procédé selon la revendication 9, dans lequel l'intégrase spécifique est l'intégrase du phage PhiC31 de *Streptomyces* et dans lequel le site de reconnaissance spécifique est un site *attB*.

11. Cellule hôte selon la revendication 6 ou procédé selon l'une quelconque des revendications 7 à 10, où le régulateur transcriptionnel codé par la cassette (a) est un régulateur transcriptionnel activable.

12. Cellule hôte ou procédé selon la revendication 11, où le régulateur transcriptionnel activable (a) est formé par le domaine de liaison à l'ADN de GAL4, le domaine de liaison au ligand du récepteur humain de la progestérone et par le domaine d'activation de NF-kappaB p65.

13. Cellule hôte selon les revendications 6, 11 ou 12, ou procédé selon les revendications 7 à 12, où l'endonucléase de restriction codée par la cassette (b) est I-SceI.

14. Cellule qui peut être produite par un procédé selon l'une quelconque des revendications 7 à 13.

15. Système pour la production d'un adénovirus de grande capacité qui comprend
(a) une cellule selon l'une quelconque des revendications 6, 11, 13 ou 14 et
(b) un adénovirus de grande capacité.

16. Procédé de production de vecteurs adénoviraux de grande capacité, qui comprend les étapes de :
(a) transfection ou infection d'une cellule hôte avec un polynucléotide qui comprend le génome de l'adénovirus de grande capacité dont la production est souhaitée ou avec un vecteur d'expression qui comprend ledit polynucléotide,
(b) culture des cellules transfectées/infectées dans des conditions adéquates pour permettre la réplication et l'encapsidation de l'adénovirus de grande capacité,
(c) récupération des particules virales de l'adénovirus de grande capacité et, facultativement,
(d) répétition des étapes (a) à (c) dans lesquelles les particules virales de grande capacité produites à l'étape (c) sont utilisées à l'étape (a) du cycle suivant,
dans lequel la cellule hôte utilisée à l'étape (a) est sélectionnée dans le groupe constitué par
(i) une cellule qui comprend
- un polynucléotide qui comprend
(a) une première cassette d'expression qui comprend une séquence nucléotidique qui code un régulateur transcriptionnel ;
(b) une deuxième cassette d'expression qui comprend une séquence nucléotidique qui code une endonucléase de restriction qui est sous le contrôle opérationnel d'une séquence régulatrice transcriptionnelle activable par le régulateur transcriptionnel (a)
- la séquence nucléotidique qui code la protéine E1A adénovirale et, facultativement,
- la protéine E1B adénovirale,
auquel cas l'étape (a) est précédée par ou réalisée simultanément à une étape de transfection avec un polynucléotide selon les revendications 1 à 4 et/ou un vecteur d'expression qui comprend ledit polynucléotide,
(ii) une cellule qui comprend un polynucléotide comprenant
(a) une première cassette d'expression qui comprend une séquence nucléotidique qui code un régulateur transcriptionnel ;
(b) une deuxième cassette d'expression qui comprend une séquence nucléotidique qui code une endonucléase de restriction qui est sous le contrôle opérationnel d'une séquence régulatrice transcriptionnelle activable par le régulateur transcriptionnel (a) et
(c) une troisième cassette d'expression qui comprend une séquence nucléotidique qui code la protéine E1A adénovirale ou les protéines E1A et E1B adénovirales, et qui est sous le contrôle opérationnel d'une séquence régulatrice transcriptionnelle activable par le régulateur transcriptionnel (a),
auquel cas l'étape (a) est précédée par ou réalisée simultanément à une étape de transfection avec un polynucléotide selon les revendications 1 à 4 et/ou un vecteur d'expression qui comprend ledit polynucléotide,
(iii) une cellule qui comprend un polynucléotide selon l'une quelconque des revendications 1 à 4, auquel cas l'étape (a) est précédée par ou réalisée simultanément à une étape de transfection avec un polynucléotide comprenant
(a) une première cassette d'expression qui comprend une séquence nucléotidique qui code un régulateur transcriptionnel ;
(b) une deuxième cassette d'expression qui comprend une séquence nucléotidique qui code une endonucléase de restriction, et qui est sous le contrôle opérationnel d'une séquence régulatrice transcriptionnelle activable par le régulateur transcriptionnel codé par le polynucléotide défini en (a) ;
et dans laquelle la cellule comprend une séquence qui code la protéine E1A adénovirale et, facultativement, la protéine E1B adénovirale,
(iv) une cellule qui comprend un polynucléotide selon l'une quelconque des revendications 1 à 4, auquel cas l'étape (a) est précédée par ou réalisée simultanément à une étape de transfection avec
- un polynucléotide comprenant
(a) une première cassette d'expression qui comprend une séquence nucléotidique qui code un régulateur transcriptionnel ;
(b) une deuxième cassette d'expression qui comprend une séquence nucléotidique qui code une endonucléase de restriction qui est sous le contrôle opérationnel d'une séquence régulatrice transcriptionnelle activable par le régulateur transcriptionnel (a) et
(c) une troisième cassette d'expression qui comprend une séquence nucléotidique qui code la protéine E1A adénovirale ou les protéines E1A et E1B adénovirales, et qui est sous le contrôle opérationnel d'une séquence régulatrice transcriptionnelle activable par le régulateur transcriptionnel (a), et/ou
- un vecteur d'expression qui comprend ledit polynucléotide,
(v) une cellule qui comprend
- un polynucléotide comprenant
(a) une première cassette d'expression qui comprend une séquence nucléotidique qui code un régulateur transcriptionnel ;
(b) une deuxième cassette d'expression qui comprend une séquence nucléotidique qui code une endonucléase de restriction, et qui est sous le contrôle opérationnel d'une séquence régulatrice transcriptionnelle activable par le régulateur transcriptionnel codé par le polynucléotide défini en (a) ;
- un polynucléotide selon les revendications 1 à 4 et
- une séquence qui code la protéine E1A adénovirale et, facultativement, la protéine E1B adénovirale, et
(vi) une cellule qui comprend
- un polynucléotide comprenant
(a) une première cassette d'expression qui comprend une séquence nucléotidique qui code un régulateur transcriptionnel ;
(b) une deuxième cassette d'expression qui comprend une séquence nucléotidique qui code une endonucléase de restriction qui est sous le contrôle opérationnel d'une séquence régulatrice transcriptionnelle activable par le régulateur transcriptionnel (a) et
(c) une troisième cassette d'expression qui comprend une séquence nucléotidique qui code la protéine E1A adénovirale ou les protéines E1A et E1B adénovirales, et qui est sous le contrôle opérationnel d'une séquence régulatrice transcriptionnelle activable par le régulateur transcriptionnel (a), et
- un polynucléotide selon l'une quelconque des revendications 1 à 4.

17. Procédé selon la revendication 16, où la culture de l'étape (b) est effectuée en présence d'un agent inducteur spécifique du régulateur transcriptionnel codé par ladite première cassette d'expression.

18. Procédé selon la revendication 17, dans lequel le régulateur transcriptionnel est formé par le domaine de liaison à l'ADN de GAL4, le domaine de liaison au ligand du récepteur humain de la progestérone et par le domaine d'activation de NF-kappaB p65 et où l'agent inducteur est une antiprogestérone synthétique, de préférence la mifépristone.

19. Procédé selon l'une quelconque des revendications 16 à 18, où le régulateur transcriptionnel codé par la cassette (a) est un régulateur transcriptionnel activable.

20. Procédé selon la revendication 19, où le régulateur transcriptionnel activable (a) est formé par le domaine de liaison à l'ADN de GAL4, le domaine de liaison au ligand du récepteur humain de la progestérone et par le domaine d'activation de NF-kappaB p65.

21. Procédé selon l'une quelconque des revendications 16 à 20, où l'endonucléase de restriction codée par la cassette (b) est I-SceI.
